(19) Europäisches Patentamt
European Patent Office
Office européen des brevets

(11) Veröffentlichungsnummer : **0 110 091**
**B1**

(12) # EUROPÄISCHE PATENTSCHRIFT

(45) Veröffentlichungstag der Patentschrift : **21.01.87**

(51) Int. Cl.⁴ : **C 07 D213/75**, A 61 K 31/44

(21) Anmeldenummer : **83110360.1**

(22) Anmeldetag : **18.10.83**

(54) **2-Amino-3-Acylamino-6-benzylamino-pyridin-Derivate mit anti-epileptischer Wirkung.**

(30) Priorität : **27.10.82 DE 3239790**

(43) Veröffentlichungstag der Anmeldung : **13.06.84 Patentblatt 84/24**

(45) Bekanntmachung des Hinweises auf die Patenterteilung : **21.01.87 Patentblatt 87/04**

(84) Benannte Vertragsstaaten : **AT BE CH DE FR GB LI LU NL SE**

(56) Entgegenhaltungen :
**BE-A- 698 384**
**BE-A- 764 362**
**CHEMISCHE ZEITUNG, 103. Jahrgang (1979), Nr. 12, Frankfurt, DE, W.v. BEBENBURG et al.: "Über substituierte Polyaminopyridine", Seiten 387-399**

(73) Patentinhaber : **Degussa Aktiengesellschaft**
**Weissfrauenstrasse 9**
**D-6000 Frankfurt am Main 1 (DE)**

(72) Erfinder : **von Bebenburg, Walter, Dr.**

**verstorben (DE)**
Erfinder : **Engel, Jürgen, Dr.**
**Cranachstrasse 25**
**D-8755 Alzenau (DE)**
Erfinder : **Heese, Joachim, Dr.**
**Fürstenbergstrasse 2**
**D-6450 Hanau 9 (DE)**
Erfinder : **Thiele, Kurt, Dr.**
**Rebbergstrasse 47 D**
**CH-4800 Zofingen (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Aus BE-A-698 384 und 764 362 sind Verbindungen der folgenden Formel bekannt :

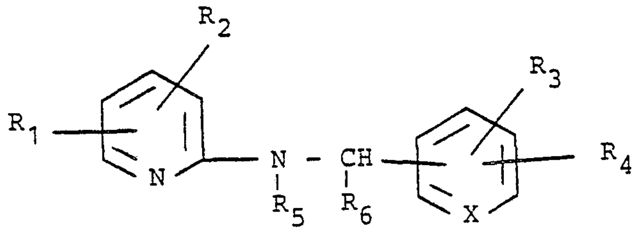

In dieser Formel bedeuten einer oder mehrere der Reste $R_1$ bis $R_4$ Aminogruppen, die acyliert oder durch niedrigmolekulare Reste alkyliert sein können, wobei diejenigen der Reste $R_1$ bis $R_4$, die keine Aminogruppen darstellen, Wasserstoff- oder Halogenatome, niedrigmolekulare Alkyl-, Trifluormethyl-, Cyan-, Rhodan-, Mercapto-, niedrigmolekulare Alkylthio-, Acylthio-, Hydroxy-, Methylendioxy-, niedridmolekulare Alkoxy-, Acyloxy-, Nitro-, Carboxy-, Carbalkoxy- oder Carbamoylgruppen bedeuten, $R_5$ ein Wasserstoffatom oder einen Acylrest, $R_6$ ein Wasserstoffatom, eine niedrigmolekulare Alkyl- oder eine Aralkylgruppe und X ein Stickstoffatom oder die CH-Gruppe darstellt und wobei die Acylreste sich von der Kohlensäure, dem Kohlensäurehalbmorpholid, von Kohlensäuremonoestern, von vorzugsweise substituierten Benzoesäuren und Pyridincarbonsäuren oder von gesättigten oder ungesättigten, gegebenenfalls durch einen Morpholinorest substituierten niedrigmolekularen aliphatischen Mono- oder Dicarbonsäuren ableiten.

Für diese Verbindungen wird eine antiphlogistische und analgetische Wirksamkeit angegeben.

Weiterhin sind aus BE-A-736 139 Verbindungen der folgenden Formel bekannt :

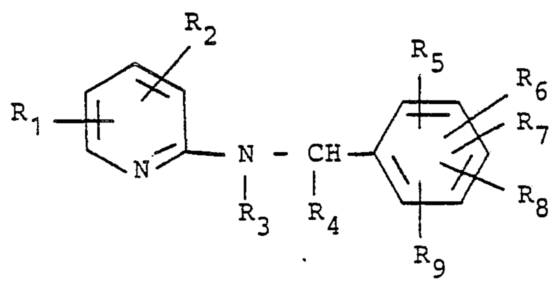

worin $R_1$ eine Aminogruppe oder eine durch niedrigmolekulare Alkylreste mit 1-6 Kohlenstoffatomen substituierte Aminogruppe bedeutet oder eine Aminogruppe darstellt, die durch Kohlensäure, durch niedridmolekulare aliphatische Kohlensäuremonoester, durch aromatische Kohlensäuremonoester, durch gegebenenfalls substituierte Benzoesäuren, durch gegebenenfalls substituierte gesättigte oder ungesättigte, gerade oder verzweigte niedrigmolekulare aliphatische Mono- oder Dicarbonsäuren mit 1-6 Kohlenstoffatomen oder durch das Kohlensäurehalbmorpholid oder durch das Kohlensäurehalbpiperidid acyliert ist, $R_2$ ein Wasserstoffatom oder die gleiche Gruppe wie $R_1$ ist, $R_3$ ein Wasserstoffatom oder die niedrigmolekulare Alkylgruppe mit 1-6 Kohlenstoffatomen oder eine Acylgruppe, wie sie für die Acylierung von $R_1$ angegeben ist, die Reste $R_5$, $R_6$, $R_7$, $R_8$ und $R_9$ gleich oder verschieden sind und Wasserstoff oder Halogenatome, Alkylgruppen mit 1-6 Kohlenstoffatomen, Trifluormethylgruppen, Hydroxygruppen, Alkoxygruppen mit 1-6 Kohlenstoffatomen, Hydroxyalkylgruppen mit 1-6 Kohlenstoffatomen, aliphatische Acylgruppen mit 1-6 Kohlenstoffatomen, Carboxygruppen oder Carboxyalkylgruppen mit 1-6 Kohlenstoffatomen und $R_4$ Wasserstoff oder eine Alkylgruppe mit 1-6 Kohlenstoffatomen oder die Gruppe

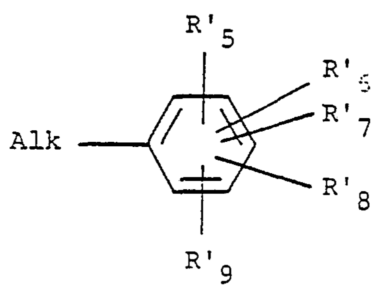

bedeuten, wobei Alk eine gegebenenfalls durch eine Hydroxygruppe oder Alkylgruppe mit 1-6 Kohlenstoffatomen oder Alkoxygruppen mit 1-6 Kohlenstoffatomen substituierte gerade oder verzweigte Alkylengruppe mit 1-3 Kohlenstoffatomen ist und die Reste $R'_5$, $R'_6$, $R'_7$, $R'_8$ und $R'_9$ gleich oder verschieden sind und die gleiche Bedeutung wie die Reste $R_5$-$R_9$ haben, wobei mindestens einer der Reste $R'_5$, $R'_6$, $R'_7$, $R'_8$ beziehungsweise $R'_9$ kein Wasserstoffatom ist, wenn Alk eine unsubstituierte Alkylengruppe ist und mindestens drei der Reste $R_5$, $R_6$, $R_7$, $R_8$ beziehungsweise $R_9$ keine Wasserstoffatome sind, wenn $R_4$ Wasserstoff oder eine Alkylgruppe bedeutet.

Auch für diese Verbindungen wird eine antiphlogistische und analgetische Wirkung angegeben.

Einen Überblick über die Herstellung und Konstitution der Verbindungen, die in den zuvor genannten Belgischen Patenten beschrieben werden, gibt auch der Artikel in der Chemiker-Zeitung *103*, Jahrgang 1979, Nr. 12, W. von Bebenburg et al : « Über substituierte Polyaminopyridine », Seiten 387 bis 399.

Die Erfindung betrifft antiepileptisch wirksame Arzneimittel sowie neue antiepileptisch wirkende Verbindungen und Verfahren zu ihrer Herstellung.

Im einzelnen betrifft die Erfindung Verbindungen der Formel

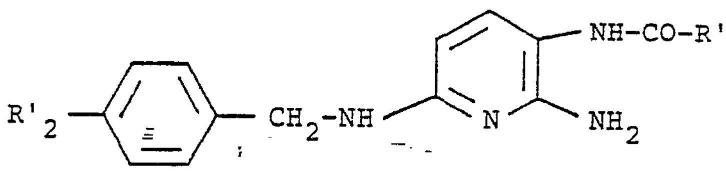

I'

worin R', eine $C_1$-$C_4$-Alkylgruppe, eine $C_1$-$C_4$-Alkoxygruppe, eine Phenoxygruppe oder eine Phenyl-$C_1$-$C_2$-alkoxygruppe bedeutet und $R'_2$ eine Trifluormethylgruppe, eine $C_1$-$C_4$-Alkylcarbonylgruppe, eine $C_1$-$C_4$-Alkylcarbonylaminogruppe oder eine Aminosulfonylgruppe ($NH_2SO_2$—) darstellt und wobei $R'_2$ auch Fluor oder Wasserstoff sein kann, falls R' eine $C_1$-$C_4$-Alkylgruppe, eine Phenoxygruppe oder eine Phenyl-$C_1$-$C_2$-alkoxygruppe ist, ferner ein antiepileptisch wirkendes Arzneimittel, enthaltend mindestens eine Verbindung der Formel I'

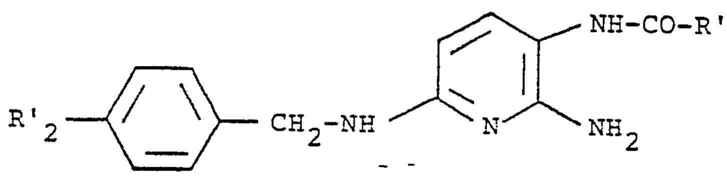

I'

worin R' eine $C_1$-$C_4$-Alkylgruppe, eine $C_1$-$C_4$-Alkoxygruppe, eine Phenoxygruppe oder eine Phenyl-$C_1$-$C_2$-alkoxygruppe bedeutet und $R'_2$ eine Trifluormethylgruppe, eine $C_1$-$C_4$-Alkylcarbonylgruppe, eine $C_1$-$C_4$-Alkylcarbonylaminogruppe oder eine Aminosulfonylgruppe ($NH_2SO_2$—) darstellt und wobei $R'_2$ auch Fluor oder Wasserstoff sein kann, falls R' eine $C_1$-$C_4$-Alkylgruppe, eine Phenoxygruppe oder eine Phenyl-$C_1$-$C_2$-alkoxygruppe ist oder deren Säureadditionssalzen und Verfahren zur Herstellung von Verbindungen gemäß Formel I dadurch gekennzeichnet, daß man

a) in einer Verbindung der Formel

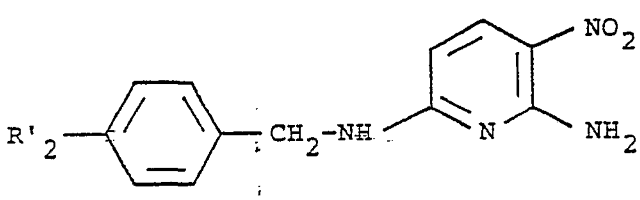

II'

worin der Rest $R'_2$ die angegebenen Bedeutungen hat, die Nitrogruppe zur Aminogruppe reduziert und in die so erhaltene 3-ständige Aminogruppe durch Acylierung den Rest R'CO— einführt, wobei R' die angegebenen Bedeutungen hat oder

b) eine Verbindung der allgemeinen Formel

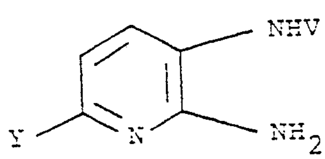

III'

mit einer Verbindung der allgemeinen Formel

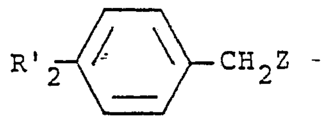

IV'

umsetzt, wobei V der Formel III' Wasserstoff oder die Gruppe —COR' ist und Y eine Aminogruppe bedeutet, falls Z ein Halogenatom, eine Aminogruppe oder die Gruppe

—OW

darstellt und wo W ein Wasserstoffatom, eine niedrigmolekulare Alkylgruppe oder eine gegebenenfalls durch Halogenatome, Nitrogruppen oder Methylgruppen substituierte Phenylgruppe ist oder Z auch eine Aminogruppe sein kann, falls Y Halogen, eine Hydroxygruppe oder die Gruppe OW ist, mit oder ohne Lösungsmittel, vorzugsweise in Gegenwart eines Kondensationsmittels bei erhöhter Temperatur umsetzt oder

c) eine Verbindung der allgemeinen Formel III', worin Y die Aminogruppe ist und V die angegebenen Bedeutungen hat, mit einer Verbindung der allgemeinen Formel

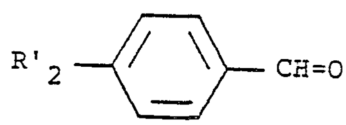

V'

unter gleichzeitiger Reduktion kondensiert und in die nach den Verfahren b) bis c) erhaltenen Verbindungen, die in 3-Stellung des Pyridinringes eine freie Aminogruppe haben, in diese 3-ständige Aminogruppe durch Acylierung den Rest R'CO— einführt und gegebenenfalls die nach den Verfahren a) bis c) erhaltenen Verbindungen in ihre Säureadditionssalze überführt.

Die erfindungsgemäßen Gegenstände umfassen außerdem die Verwendung von Verbindungen der Formel I

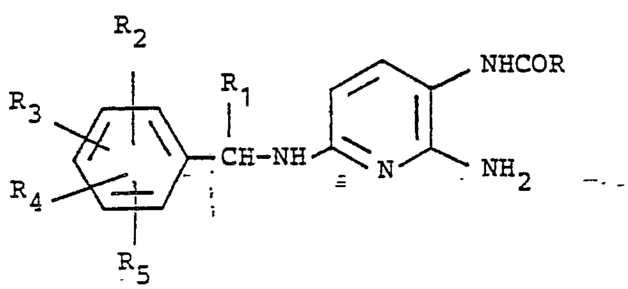

(I)

worin R eine $C_1$-$C_4$-Alkylgruppe, eine $C_1$-$C_4$-Alkoxygruppe, eine Phenoxygruppe oder eine Phenyl-$C_1$-$C_2$-alkoxygruppe, $R_1$ Wasserstoff oder eine $C_1$-$C_4$-Alkylgruppe und $R_5$ Wasserstoff oder eine $C_1$-$C_4$-Alkylgruppe bedeutet und die Reste $R_2$, $R_3$ und $R_4$ gleich oder verschieden sind und Wasserstoff, Trifluormethyl, Halogenatome, $C_1$-$C_4$-Alkylgruppen, $C_1$-$C_4$-Alkylcarbonylgruppen, die Aminosulfonylgruppe ($NH_2$—$SO_2$—) oder eine $C_1$-$C_4$-Alkylcarbonylaminogruppe bedeuten und deren Säureadditionssalzen für die Herstellung eines Arzneimittels zur Behandlung von Epilepsie.

Die Verbindungen gemäß den Stoffansprüchen 1-5 sowie den Verwendungsansprüchen 10 bis 15 besitzen eine ausgeprägte antiepileptische Wirkung mit langer Halbwertszeit in epileptischen Patienten (zum Beispiel 10 Stunden). Diese Wirkung ist sowohl im Hinblick auf den eingangs erwähnten vorbekannten Stand der Technik, insbesondere aufgrund der Tatsache, daß daraus keine Antiepileptika bekannt sind, die einen Pyridin-Ring oder eine Benzylamino-Gruppe enthalten, überraschend.

Die in der Formel I des Verwendungsanspruchs 10 vorkommenden Reste $R_2$, $R_3$, $R_4$ und $R_5$ können jeweils gleich oder verschieden sein. Falls die Reste $R_2$, $R_3$, $R_4$ und $R_5$ Alkylgruppen oder Alkylcarbonylaminogruppen bedeuten, können die Alkylreste gerade oder verzweigt sein. Dasselbe gilt hinsichtlich der Reste R und $R_1$, falls diese Alkyl- beziehungsweise Alkoxygruppen darstellen. Falls die Reste $R_2$, $R_3$ und/oder $R_4$ Halogenatome bedeuten, handelt es sich vorzugsweise um Fluor und Chlor. Falls R eine Phenyl-$C_1$-$C_2$-alkoxygruppe darstellt, handelt es sich insbesondere um eine Ethoxygruppe, die in β-Stellung (das heißt in 2-Stellung) durch den Phenylrest substituiert ist (2-Phenyl-ethyl-(1)-oxygruppe). Die vorkommenden Alkylgruppen sowie Alkoxygruppen bestehen insbesondere aus einem oder zwei C-Atomen, das heißt es handelt sich bei den Resten R, sowie $R_1$ bis $R_5$, falls diese Alkylgruppen, Alkoxygruppen oder Alkylcarbonylaminogruppen darstellen, vorzugsweise um Methyl-, Ethyl-, Methoxy- und Ethoxyreste. Falls der Phenylrest (der die Reste $R_2$ bis $R_5$ enthält) einen Substituenten enthält und

dieser Substituent Halogen, $CF_3$, $C_1$-$C_4$-Alkylcarbonyl, $C_1$-$C_4$-Alkylcarbonylamino oder Aminosulfonyl ist, sitzt dieser vorzugsweise in 4- oder 3-Stellung des Phenylringes ; dies gilt auch, wenn dieser Phenylring zusätzliche Alkylsubstituenten enthält. Alkylsubstituenten des vorstehend erwähnten Phenylrestes sitzen vorzugsweise in den 2-Stellungen und/oder der 4-Stellung des Phenylringes.

Beispiele für Verbindungen der Formel I mit antiepileptischer Wirkung sind :

2-Amino-3-carbethoxyamino-6-benzylamino-pyridin ;
2-Amino-3-carbethoxyamino-6-(4-ethyl-benzylamino)-pyridin ;
2-Amino-3-carbethoxyamino-6-(3,5-dimethyl-benzylamino)-pyridin ;
2-Amino-3-carbethoxyamino-6-(4-acetyl-benzylamino)-pyridin ;
2-Amino-3-carbethoxyamino-6-(α-methyl-4-fluor-benzylamino)-pyridin ;
2-Amino-3-carbethoxyamino-6-(4-fluor-benzylamino)-pyridin ;
2-Amino-3-carbethoxyamino-6-(3-fluor-benzylamino)-pyridin ;
2-Amino-3-carbethoxyamino-6-(4-chlor-benzylamino)-pyridin ;
2-Amino-3-carbethoxyamino-6-(4-aminosulfonyl-benzylamino)-pyridin ;
2-Amino-3-carbethoxyamino-6-(4-trifluormethyl-benzylamino)-pyridin ;
2-Amino-3-carbethoxyamino-6-(4-methylcarbonylamino-benzylamino)-pyridin ;
2-Amino-3-carbethoxyamino-6-(2,4,6-trimethyl-benzylamino)-pyridin ;
2-Amino-3-carbethoxyamino-6-(3,4,6-trimethyl-benzylamino)-pyridin ;
2-Amino-3-carbethoxyamino-6-(2,3,4-trimethyl-benzylamino)-pyridin ;
2-Amino-3-carbethoxyamino-6-(2,4,6-triethyl-benzylamino)-pyridin ;
2-Amino-3-carbethoxyamino-6-(2,6-dimethyl-4-ethyl-benzylamino)-pyridin ;
2-Amino-3-carbethoxyamino-6-(2-fluor-4-methyl-benzylamino)-pyridin ;
2-Amino-3-carbethoxyamino-6-(2,4-dimethyl-5-chlor-benzylamino)-pyridin ;
2-Amino-3-carbethoxyamino-6-(2,4-dimethyl-5-fluor-benzylamino)-pyridin ;
2-Amino-3-carbethoxyamino-6-(2,4,6-trimethyl-3-fluor-benzylamino)-pyridin ;
2-Amino-3-acetylamino-6-(4-fluor-benzylamino)-pyridin ;
2-Amino-3-phenoxycarbonylamino-6-benzylamino-pyridin ;
2-Amino-3-phenoxycarbonylamino-6-(4-fluor-benzylamino)-pyridin ;
2-Amino-3-phenethoxycarbonylamino-6-benzylamino-pyridin ;
2-Amino-3-phenethoxycarbonylamino-6-(3-trifluormethyl-benzylamino)-pyridin.

Die antiepileptische Wirkung der Verbindungen der Formel I gemäß dem Verwendungsanspruch 10 sowie dem Stoffanspruch 1 ist im Tierversuch als antikonvulsive Wirkung meßbar. Die Verbindungen I und I' zeigen daher am maximalen Elektroschock an der Maus eine gute antikonvulsive Wirkung.

Beispielsweise tritt bei obengenannter Versuchsmethode bei einer Dosis von 40 mg/Körpergewicht der Maus die antikonvulsive Wirkung auf. Diese antikonvulsive Wirkung ist mit der Wirkung des bekannten Arzneimittels « Valproate » (Valproinsäure-Salz) vergleichbar. Die niedrigste, bereits wirksame Dosis in dem obenangegebenen Tierversuch ist beispielsweise :

0,2 mg/kg oral
0,05 mg/kg intravenös.

Als allgemeiner Dosisbereich für die antikonvulsive Wirkung bei der Maus kommt beispielsweise in Frage :

0,2-2 000 mg/kg oral, insbesondere 40 mg/kg,
0,05-500 mg/kg intravenös, insbesondere 10 mg/kg.

Indikationen für die die erfindungsgemäßen Verbindungen in Betracht kommen können : Epilepsie.

Im einzelnen geht die antikonvulsive Wirkung der Verbindungen der Formel I des Verwendungsanspruchs 10 sowie der Verbindungen I' des Stoffanspruchs beispielsweise aus der folgenden Tabelle hervor :

(Siehe Tabelle 1 Seite 6 f.)

Fortsetzung Tabelle 1 :

Die Zahlen hinter dem Schrägstrich in den Spalten 3 und 4 bedeuten jeweils den Quotienten TD 50/ED 50. Sie geben also die therapeutische Breite bezüglich der unerwünschten neurotoxischen

Tabelle 1

Antikonvulsive Wirkung von Verbindungen der Formel I bei der Maus ; ausgedrückt als ED 50 in mg/kg bei intraperitonealer Applikation

| Verbindung Chiffre-Nummer | TD 50 | maximaler Elektroschock (MES) ED 50 | Metrazol-Test (Met) ED 50 |
|---|---|---|---|
| D 7175 | 45.6 | 19.7/2.3 | 9.46/4.8 |
| D 10 328 | 1.09 | 1.97/0.55 | 1.01/1.08 |
| D 10 981 | 32.7 | 17.2/1.90 | 9.07/3.61 |
| D 11 208 | 76.3 | 27.9/2.73 | 11.2/6.81 |
| D 9663 | 142.3 | 123.8/1.15 | 57.2/2.49 |
| D 9389 | 23.9 | 14.6/1.64 | 10.0/2.39 |
| D 9998 | 37.7 | 22.5/1.67 | 20.3/1.86 |
| D 13 223 | 201.0 | 84.3/2.38 | 93.8/2.14 |
| D 10 558 | 73.9 | 53.8/1.37 | 18.4/4.0 |
| D 9805 | 529.6 | 107.7/4.9 | 549.8/0.96 |

TD 50 : ist die ED 50 für das Verhalten der Mäuse am Versuchsmodell des rotierenden Stabes in mg/kg bei intraperitonealer Applikation. Die TD 50 ist ein Maß für die unerwünschte neurotoxische Nebenwirkung.

Nebenwirkung (gemessen am Modell des rotierenden Stabes) an.

Verbindungen gemäß den Chiffre-Nummer :
D 7 175 : 2-Amino-3-carbethoxyamino-6-benzylamino-pyridin-hydrochlorid ;
D 10 328 : 2-Amino-3-carbethoxyamino-6-(2,4,6-trimethyl-benzylamino)-pyridin-hydrochlorid ;
D 10 981 : 2-Amino-3-carbethoxyamino-6-(3-fluor-benzylamino)-pyridin-hydrochlorid ;
D 11 208 : 2-Amino-3-carbethoxyamino-6-(4-trifluormethyl-benzylamino)-pyridin-hydrochlorid ;
D 9 663 : 2-Amino-3-carbethoxyamino-6-(4-methylcarbonylamino-benzylamino)-pyridin-hydrochlorid ;
D 9 389 : 2-Amino-3-carbethoxyamino-6-(4-chlor-benzylamino)-pyridin-hydrochlorid ;
D 9 998 : 2-Amino-3-carbethoxyamino-6-(4-fluor-benzylamino)-pyridin-hydrochlorid ;
D 13 223 : 2-Amino-3-acetamino-6-(4-fluor-benzylamino)-pyridin-hydrochlorid ;
D 10 558 : 2-Amino-3-carbethoxyamino-6-(4-methylcarbonyl-benzylamino)-pyridin ;
D 9 805 : 2-Amino-3-carbethoxyamino-6-(4-aminosufonyl-benzylamino)-pyridin.

Beschreibung der Versuchsmethoden zur Prüfung der antikonvulsiven Wirkung :

Alle Versuche wurden mit männlichen Carworth Farms-Mäusen durchgeführt.

Die Substanzen wurden in drei Dosen (30, 100, 300 mg/kg) getestet (600 mg/kg wurde mitgeprüft, wenn eine ausreichende Substanzmenge vorhanden war). Die Substanzen wurden jeweils in 30 %iger wässriger Polyethylenglykol-Lösung gelöst.

1. MES — maximaler Elektroschock-Anfalls-Test

Ein maximaler Elektroschock-Anfall wird erreicht durch Wechselstrom (60 Hz, 50 mA Intensität), mit dem die Maus 0,2 Sekunden lang über Corneal-Elektroden gereizt wird. Eine 5-7 mal schwächere Reizintensität reicht aus, um einen minimalen Elektroschock-Anfall zu erzeugen. Ein Tropfen 0,9 % NaCl-Salzlösung wird vor dem Anbringen der Elektroden ins Auge gegeben, um den Tod der Tiere vorzubeugen. Die Testsubstanzen werden vor der Reizung mit dem Wechselstrom intraperitoneal verabreicht. Aufhebung der tonischen Streckungskomponente des Anfalls (hintere Extremitäten) wird als Schutzwirkung angesehen. Die Ergebnisse werden wie folgt dargestellt :

Anzahl der Tiere mit Schutzwirkung/Gesamtzahl der Testtiere.

2. s. c. Met — Subcutaner Pentetrazol-Anfalls-Schwellen-Test

85 mg/kg Pentetrazol (ruft in mehr als 95 % der Mäuse Anfälle hervor) wird subcutan als 0,5 % Lösung appliziert (unter die Nackenhaut). Das Tier wird 30 Minuten lang beobachtet. Das Ausbleiben eines deutlich wahrnehmbaren Anfalls (einmaliges Auftreten von chronischen Spasmen von 5 Sekunden Dauer) aufgrund der vorhergehenden intraperitonealen Applikation der Testsubstanzen wird als Schutzwirkung gewertet (die Testsubstanzen werden vor der Pentetrazol-Applikation intraperitoneal appliziert). Die Ergebnisse werden wie folgt dargestellt :

Anzahl der Tiere mit Schutzwirkung/Gesamtzahl der Testtiere.

3. TD 50 — neurotoxische Wirkung

Der Test am rotierenden Stab wird zur Beurteilung der Neurotoxizität herangezogen. Die Tiere werden auf einen rotierenden (6 Umdrehungen pro Minute) Plastikstab mit einem Durchmesser von 2,54 cm gesetzt. Normale unbehandelte Mäuse können unbegrenzt auf dem mit dieser Geschwindigkeit rotierenden Stab verbleiben. Als neurologisch toxisch wird ein Tier bezeichnet, wenn es innerhalb einer Minute vom rotierenden Stab fällt.

Die Ergebnisse werden wie folgt dargestellt :

Anzahl der Tiere die fallen/Gesamtzahl der Testtiere.

Beispiele für die Toxizität an der Maus per os (ausgedrückt als LD 50 in mg/kg) sind die folgenden :

| Chiffre | LD 50/Maus per os |
|---|---|
| D 7 175 | 402 |
| D 10 328 | 127 |
| D 10 981 | 2 061 |
| D 9 389 | 1 250-1 500 |
| D 9 998 | 617 |

Die Bestimmung der oralen Toxizität an der weissen Maus erfolgte in der internationalen Versuchsanordnung nach Miller & Tainter (Proc. Soc. Exper. Biol. a. Med. 57, 261 (1944) bei einer Beobachtungszeit von 24 Stunden. Die Toxizität wird als LD 50 in mg/kg angegeben. Die LD 50 ist diejenige Dosis, die bei 50 % der eingesetzten Tiere zum Tode führt.

Die pharmazeutischen Zubereitungen können im allgemeinen zwischen 0,2 bis 2 000, vorzugsweise 40 bis 300 mg der erfindungsgemäßen aktiven Komponente(n) enthalten.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage : Ölige oder alkoholische beziehungsweise wässrige Lösungen sowie Suspensionen und Emulsionen. Bevorzugte Anwendungsformen sind Tabletten, die zwischen 0,2 und 2 000 mg oder Lösungen, die zwischen 0,5 bis 30 % an aktiver Substanz enthalten.

Die Einzeldosis der erfindungsgemäßen aktiven Komponenten kann beispielsweise liegen :

a) bei oralen Arzneiformen zwischen 0,2 bis 2 000 mg, vorzugsweise 10 bis 300 mg, insbesondere 40 bis 200 mg ;

b) bei parenteralen Arzneiformen (zum Beispiel intravenös, intramuskulär) zwischen 0,05 bis 500 mg, vorzugsweise 10 mg ;

c) bei Arzneiformen zur rektalen oder vaginalen Applikation zwischen 0,5 bis 5 000, vorzugsweise 50 mg.

Die Dosen sind jeweils bezogen auf die freie Base.

Beispielsweise können 3 mal täglich 1 bis 4 Tabletten mit einem Gehalt von 0,2 bis 2 000 mg wirksamer Substanz oder zum Beispiel bei intravenöser Injektion 1 bis 4 mal täglich eine Ampulle von 1 bis 5 ml Inhalt mit 5 bis 1 500 mg Substanz empfohlen werden. Bei oraler Verabreichung ist die minimale tägliche Dosis beispielsweise 10 mg ; die maximale tägliche Dosis bei oraler Verabreichung soll nicht über 4 000 mg liegen.

Beispielsweise können Dosierungen gemäß Tabelle 2 empfohlen werden.

Tabelle 2

| Verbindung | Einzeldosis in mg, kann beispielsweise 1- bis 3-mal täglich verabreicht werden | maximale Tagesdosis in mg |
|---|---|---|
| D 9998 | 100 - 300 | 800 |
| D 7175 | 100 - 300 | 800 |
| D 10 328 | 10 - 50 | 150 |
| D 10 981 | 100 - 300 | 2000 |
| D 11 208 | 100 - 300 | 800 |
| D 9663 | 300 - 500 | 1000 |
| D 9389 | 80 - 200 | 1500 |
| D 13 223 | 200 - 600 | 3000 |
| D 10 558 | 100 - 300 | 800 |
| D 9805 | 200 - 400 | 3000 |

Die akute Toxizität der erfindungsgemäßen Verbindungen an der Maus (ausgedrückt durch die LD 50 mg/kg ; Methode nach Miller und Tainter : Proc. Soc. Exper. Biol. a. Med. *57* (1944) 261) liegt beispielsweise bei oraler Applikation zwischen 10 und 10 000 mg/kg (beziehungsweise oberhalb 10 000 mg/kg).

Die Verbindungen die durch die Formel I des Verwendungsanspruchs 10 sowie den Stoffanspruch 1 definiert sind, sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der Verbindungen der Formel I, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche Stoffe in Frage, die in folgenden

Literaturstellen als Hilfsstoffe für Pharmazie, Kosmetik und angrenzende Gebiete empfohlen beziehungsweise angegeben sind : Ullmanns Encyklopädie der technischen Chemie, Band 4 (1953), Seite 1 bis 39 ; Journal of Pharmaceutical Sciences, Band 52 (1963), Seite 918 u. ff., H. v. Czetsch-Lindenwald, Hilfsstoffe für Pharmazie und angrenzende Gebiete ; Pharm. Ind., Heft 2, 1961, Seite 72 u. ff. ; Dr. H. P. Fiedler, Lexikon der Hilfsstoffe für Pharmazie, Kosmetik und angrenzende Gebiete Cantor KG. Aulendorf in Württemberg 1971.

Beispiele hierfür sind Gelatine, natürliche Zucker wie Rohrzucker oder Milchzucker, Lecithin, Pektin, Stärke (zum Beispiel Maisstärke), Alginsäure, Tylose, Talkum, Lycopodium, Kieselsäure (zum Beispiel kolloidale), Cellulose, Cellulosederivate (zum Beispiel Celluloseäther, bei denen die Cellulose-Hydroxygruppen teilweise mit niederen gesättigten aliphatischen Alkoholen und/oder niederen gesättigten aliphatischen Oxyalkoholen veräthert sind, zum Beispiel Methyloxypropylcellulose), Stearate, Magnesium- und Calciumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnussöl, Rhizinusöl, Olivenöl, Sesamöl, Baumwollsaatöl, Maisöl, Weizenkeimöl, Sonnenblumensamenöl, Kabeljau-Leberöl, Mono-, Di- und Triglyceride von gesättigten Fettsäuren $C_{12}H_{24}O_2$ bis $C_{18}H_{36}O_2$ und deren Gemische), pharmazeutisch verträgliche ein- oder mehrwertige Alkohole und Polyglykole wie Polyäthylenglykole sowie Derivate hiervon, Ester von aliphatischen gesättigten oder ungesättigten Fettsäuren (2 bis 22 C-Atome, insbesondere 10 bis 18 C-Atome) mit einwertigen aliphatischen Alkoholen (1 bis 20 C-Atome) oder mehrwertigen Alkoholen wie Glykolen, Glycerin, Diäthylenglykol, Pentaerythrit, Sorbit, Mannit und so weiter, die gegebenenfalls auch veräthert sein können, Benzylbenzoat, Dioxolane, Glyzerinformale, Tetrahydrofurfurylalkohol, Polyglycoläther mit $C_1$-$C_{12}$-Alkoholen, Dimethylacetamid, Lactamide, Lactate, Äthylcarbonate, Silicone (insbesondere mittelviskose Dimethylpolysiloxane) Magnesiumcarbonat und ähnliche.

Zur Herstellung von Lösungen kommen beispielsweise Wasser oder physiologisch verträgliche organische Lösungsmittel in Frage, wie zum Beispiel Äthanol, 1,2-Propylenglykol, Polyglykole und deren Derivate, Dimethylsulfoxyd, Fettalkohole, Triglyceride, Partialester des Glycerins, Paraffine und ähnliche.

Bei der Herstellung der Zubereitungen können bekannte und übliche Lösungsvermittler, beziehungsweise Emulgatoren, verwendet werden. Als Lösungsvermittler und Emulgatoren kommen beispielsweise in Frage : Polyvinylpyrrolidon, Sorbitanfettsäureester wie Sorbitantrioleat, Lecithin, Acacia, Tragacanth, polyoxyäthyliertes Sorbitanmonooleat, polyoxyäthylierte Fette, polyoxyäthylierte Oleotriglyceride, linolisierte Oleotriglyceride, Polyäthylenoxyd-Kondensationsprodukte von Fettalkoholen, Alkylphenolen oder Fettsäuren oder auch 1-Methyl-3-(2-hydroxyäthyl)-imidazolidon-(2). Polyoxyäthyliert bedeutet hierbei, daß die betreffenden Stoffe Polyoxyäthylenketten enthalten, deren Polymerisationsgrad im allgemeinen zwischen 2 bis 40 und insbesondere zwischen 10 bis 20 liegt.

Solche polyoxyäthylierten Stoffe können beispielsweise durch Umsetzung von hydroxylgruppenhaltigen Verbindungen (beispielsweise Mono- oder Diglyceride oder ungesättigte Verbindungen wie zum Beispiel solchen die Ölsäurereste enthalten) mit Äthylenoxyd erhalten werden (zum Beispiel 40 Mol Äthylenoxyd pro Mol Glycerid).

Beispiele für Oleotriglyceride sind Olivenöl, Erdnussöl, Rhizinusöl, Sesamöl, Baumwollsaatöl, Maisöl (siehe auch Dr. H. P. Fiedler « Lexikon der Hilfsstoffe für Pharmazie, Kosmetik und angrenzende Gebiete » 1971, Seite 191 bis 195).

Darüberhinaus ist der Zusatz von Konservierungsmitteln, Stabilisatoren, Puffersubstanzen, zum Beispiel Calciumhydrogenphosphat, kolloidales Aluminiumhydroxyd, Geschmackskorrigentien, Antioxydantien und Komplexbildnern (zum Beispiel Äthylendiaminotetraessigsäure) und dergleichen möglich. Gegebenenfalls ist zur Stabilisierung des Wirkstoffmoleküls mit physiologisch verträglichen Säuren oder Puffern auf einen pH-Bereich von ca. 3 bis 7 einzustellen. Im allgemeinen wird ein möglichst neutraler bis schwach saurer (bis pH 5) pH-Wert bevorzugt.

Als Antioxydantien kommen beispielsweise Natriummetabisulfit, Ascorbinsäure, Gallussäure, Gallussäure-alkylester, Butylhydroxyanisol, Nordihydroguajaretsäure, Tocopherole sowie Tocopherole + Synergisten (Stoffe die Schwermetalle durch Komplexbildung binden, beispielsweise Lecithin, Ascorbinsäure, Phosphorsäure) zur Anwendung. Der Zusatz der Synergisten steigert die antioxygene Wirkung der Tocopherole erheblich.

Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid und Formalinderivate in Betracht.

Die pharmazeutische und galenische Handhabung der erfindungsgemäßen Verbindungen erfolgt nach den üblichen Standardmethoden. Beispielsweise werden Wirkstoff(e) und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80 °C, vorzugsweise 20 bis 50 °C, insbesondere bei Raumtemperatur gearbeitet wird. Im übrigen wird auf das folgende Standardwerk verwiesen : Sucker, Fuchs, Speiser, Pharmazeutische Technologie, Thieme-Verlag Stuttgart, 1978.

Die Applikation der Wirkstoffe beziehungsweise der Arzneimittel kann auf die Haut oder Schleimhaut oder in das Körperinnere erfolgen, beispielsweise oral, enteral, pulmonal, rectal, nasal, vaginal, lingual, intravenös, intraarteriell, intrakardial, intramuskulär, intraperitoneal, intracutan, subcutan.

Insbesondere ist auch der Zusatz anderer Arzneimittelwirkstoffe möglich beziehungsweise günstig.

Die Herstellung der Verbindungen der Formel I kann gemäß BE-A-698 384, 764 362 und 736 139 erfolgen beziehungsweise analog zu den dort angegebenen Verfahren. In den Beispielen ist die Herstellung einiger Verbindungen beschrieben. Natürlich kann die Herstellung auch nach dem Verfahren von Anspruch 9 erfolgen.

Je nach den Verfahrensbedingungen und Ausgangsstoffen erhält man die erfindungsgemäßen Verbindungen der Formel I in freier Form oder in Form ihrer Salze. Diese Salze können in an sich bekannter Weise, beispielsweise mit Alkali oder Ionenaustauschern, wieder in die freie Base übergeführt werden. Von letzterer lassen sich durch Umsetzung mit organischen oder anorganischen Säuren wieder die Salze gewinnen. Als solche Säuren seien beispielsweise genannt: Halogenwasserstoffsäuren, Schwefelsäure, Phosphorsäuren, Salpetersäure, Perchlorsäure, organische Mono-, Di- oder Tricarbonsäuren der aliphatischen, alicyclischen, aromatischen oder heterocyclischen Reihe sowie Sulfonsäuren. Beispiele hierfür sind: Ameisen-, Essig-, Propion-, Bernstein-, Glykol-, Milch-, Äpfel-, Wein-, Zitronen-, Ascorbin-, Malein-, Fumar-, Hydroxymalein- oder Brenztraubensäure; Phenylessig-, Benzoe-, p-Aminobenzoe-, Anthranil-, p-Hydroxy-benzoe-, Salicyl- oder p-Aminosalicylsäure, Embonsäure, Methansulfon-, Äthansulfon-, Hydroxyäthansulfon-, Äthylensulfonsäure; Halogenbenzolsulfon-, Toluolsulfon-, Naphthalinsulfonsäure oder Sulfanilsäure.

Die Überführung eines Salzes einer Verbindung der Formel I in ein anderes Salz ist beispielsweise in Beispiel 10 beschrieben.

Die erhaltenen Verbindungen, die optisch aktive Kohlenstoffatome enthalten und in der Regel als Racemate anfallen, können in an sich bekannter Weise zum Beispiel mittels einer optisch aktiven Säure in die optisch aktiven Isomeren gespalten werden.

Es ist aber auch möglich, von vornherein optisch aktive beziehungsweise auch diastereomere Ausgangsstoffe einzusetzen, wobei dann als Endprodukt eine entsprechende reine optisch aktive Form beziehungsweise diastereomere Konfiguration erhalten wird.

Die Erfindung betrifft auch eine neue Verbindungsgruppe der Formel I' gemäß den Stoffansprüchen 1-5. Diese Verbindungen der Formel I' stellen eine kleinere Verbindungsgruppe innerhalb der Verbindungsgruppe der Formel I (Verwendungsanspruch 10) dar, wobei es sich im Gegensatz zu den Verbindungen der Formel I, von denen eine größere Zahl bereits bekannte Verbindungen sind, sämtlich um neue Verbindungen handelt. Die Verbindungen der Formel I' des Stoffanspruchs 1 besitzen selbstverständlich ebenfalls die ausgeprägte antiepileptische Wirkung mit langer Halbwertszeit in epileptischen Patienten (zum Beispiel 10 Stunden). Diese Wirkung ist insbesondere im Hinblick auf den eingangs erwähnten vorbekannten Stand der Technik überraschend.

Falls der Rest $R'_2$ der Formel I' eine Alkylcarbonylgruppe oder Alkylcarbonylaminogruppe bedeutet, können die Alkylreste gerade oder verzweigt sein. Dasselbe gilt hinsichtlich des Restes R' der Formel I, falls dieser einen Alkyl- beziehungsweise Alkoxyrest darstellt. Falls R' eine Phenyl-$C_1$-$C_2$-alkoxygruppe darstellt, handelt es sich insbesondere um eine Ethoxygruppe, die in ß-Stellung (das heißt in 2-Stellung) durch den Phenylrest substituiert ist (2-Phenyl-ethyl-(1)-oxy-gruppe). Die vorkommenden Alkylgruppen sowie Alkoxygruppen bestehen insbesondere aus einem oder zwei C-Atomen, das heißt es handelt sich bei den Resten R', sowie $R'_2$, falls diese Alkylgruppen, Alkoxygruppen, Alkylcarbonylgruppen oder Alkylcarbonylaminogruppen darstellen, vorzugsweise um Methyl-, Ethyl-, Methoxy- und Ethoxyreste.

Beispiele für die neuen Verbindungen der Formel I' mit antiepileptischer Wirkung sind:

2-Amino-3-carbethoxyamino-6-(4-acetyl-benzylamino)-pyridin;
2-Amino-3-carbethoxyamino-6-(4-aminosulfonyl-benzylamino)-pyridin;
2-Amino-3-carbethoxyamino-6-(4-trifluormethyl-benzylamino)-pyridin;
2-Amino-3-carbethoxyamino-6-(4-methylcarbonylamino-benzyl-amino)-pyridin;
2-Amino-3-acetylamino-6-(4-fluor-benzylamino)-pyridin;
2-Amino-3-phenoxycarbonylamino-6-benzylamino-pyridin;
2-Amino-3-phenoxycarbonylamino-6-(4-fluor-benzylamino)-pyridin;
2-Amino-3-phenethoxycarbonylamino-6-benzylamino-pyridin;
2-Amino-3-phenethoxycarbonylamino-6-(3-trifluormethyl-benzylamino)-pyridin.

Für die Verbindungen der Formel I' (siehe Anspruch 1) liegt die Toxizität an der Maus *per os* (ausgedrückt als LD 50 in mg/kg) beispielsweise zwischen 100-2 000.

Zu dem Verfahren a) von Anspruch 9

Für die Reduktion entsprechend dem Verfahren a) hat sich als besonders geeignet die katalytische Hydrierung erwiesen. Als Katalysatoren kommen zum Beispiel in Frage: Raney-Nickel, Edelmetalle wie Palladium und Platin sowie Verbindungen davon, mit und ohne Träger, wie beispielsweise Bariumsulfat, Calciumsulfat und so weiter. Es empfiehlt sich, die Hydrierung der Nitrogruppe bei Temperaturen zwischen 20 und 100 °C und einem Druck von ungefähr 1 bis 70 bar in einem Lösungsmittel vorzunehmen. Als Lösungsmittel eignen sich beispielsweise $C_1$-$C_4$-Alkanole, cyclische Ether wie Dioxan, Tetrahydrofuran, Methoxy-ethanol, Wasser, aromatische Kohlenwasserstoffe (Benzol, Toluole, Xylole) sowie Gemische dieser Mittel. Für die anschließende Isolierung der reduzierten Verbindungen kann es in manchen Fällen von Vorteil sein, wenn zu Beginn dem zu hydrierenden Gemisch Trockenmittel, wie wasserfreies Natrium- oder Magnesiumsulfat zugesetzt werden.

Die Reduktion kann aber auch mit nascierendem Wasserstoff beispielsweise Zink/Salzsäure, Zinn/Salzsäure, Eisen/Salzsäure oder mit Salzen des Schwefelwasserstoffs in Alkohol/Wasser bei etwa 70 bis etwa 120 °C oder mit aktiviertem Aluminium in wasserhaltigem Äther bei 20 bis 40 °C oder mit Zinn(II)-Chlorid/Salzsäure durchgeführt werden.

Falls eine Ausgangsverbindung eingesetzt wird, die eine Oxogruppe enthält (zum Beispiel Alkylcarbonyl) kann es zweckmäßig sein, diese Oxogruppe durch übliche Acetalbildung (zum Beispiel in Form des Ethylenacetals) zu schützen. Dies gilt insbesondere für die katalytische Hydrierung.

Das so erhaltene Reaktionsprodukt wird zweckmäßig sofort in der anfallenden Reaktionsmischung mit einer Verbindung umgesetzt, die geeignet ist, ein Wasserstoffatom der durch die Reduktion erhaltenen 3-ständigen Aminogruppe durch die Gruppe —COR′ zu ersetzen, ohne daß das 2,3-Diamino-6-benzylamino-pyridin-derivat isoliert werden muß. Insbesondere gilt dies für den Fall der katalytischen Hydrierung. Selbstverständlich kann diese zuletzt genannte Verbindung auch isoliert werden und dann die R′CO-Gruppe eingeführt werden. Die Einführung der R′CO-Gruppe kann in der hierfür üblichen Weise mit den hierfür üblichen Reagenzien erfolgen. Beispiele für solche Reagenzien sind : Halogenameisensäureethylester, wie Chlor-, Brom- oder Jodameisensäureethylester, -phenylester oder -phenyl-$C_1$-$C_2$-alkylester. Falls R′ eine $C_1$-$C_4$-Alkylgruppe ist, kommen als Acyleringsmittel zum Beispiel die Halogenide (Chloride, Bromide, Jodide) oder Anhydride der $C_1$-$C_4$-Alkancarbonsäuren in Frage. Da die freien Amine der Formel I′, worin die Gruppe —COR′ Wasserstoff ist, sauerstoffempfindlich sind, wird zweckmäßig unter Stickstoffatmosphäre gearbeitet.

Die Einführung der R′CO-Gruppe wird im allgemeinen in einem inerten Lösungs- oder Suspensionsmittel bei Temperaturen zwischen 0 bis 60 °C, insbesondere 5 bis 40 °C, vorzugsweise 20 bis 25 °C durchgeführt. Als Lösungsmittel kommen beispielsweise in Betracht : Gesättigte alicyclische und cyclische Ether (Dioxan, Tetrahydrofuran, niedere Dialkylether wie Diethylether, Diisopropylether), niedere Alkanole wie Ethanol, Isopropanol, Butanol, niedere aliphatische Ketone (Aceton, Methylethylketon), niedere aliphatische Kohlenwasserstoffe oder Halogenkohlenwasserstoffe (Methylenchlorid, Chloroform, 1,2-Dichlorethan), aromatische Kohlenwasserstoffe (Benzol, Toluol, Xylol), niedere Dialkylamide von niederen gesättigten aliphatischen Carbonsäuren (Dimethylformamid, Dimethylacetamid), Tetramethylharnstoff, N-Methylpyrrolidon, Dimethylsulfoxid beziehungsweise Mischungen dieser Mittel.

Im allgemeinen werden die Reaktionskomponenten in molaren Mengen umgesetzt. Gegebenenfalls kann es jedoch zweckmäßig sein, eine Reaktionskomponente in leichtem Überschuß einzusetzen. Gegebenenfalls kann die Umsetzung auch in Gegenwart von basischen beziehungsweise säurebindenden Mitteln, wie Alkalicarbonaten (Pottasche, Soda), Alkalihydrogencarbonaten, Alkaliacetaten, Alkalihydroxyden oder tertiären Aminen (beispielsweise Triethylamin) durchgeführt werden. Letzteres gilt insbesondere, wenn Halogenameisensäureester eingesetzt werden.

Die Ausgangsstoffe der Formel II′ sind bekannt oder werden durch Umsetzung von 2-Amino-3-nitro-6-chlorpyridin mit Aminen der Formel

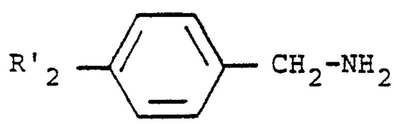

mit oder ohne Lösungsmittel bei Temperaturen zwischen 0 bis 200 °C gegebenenfalls in Gegenwart eines zusätzlichen Salzsäureakzeptors analog der in den BE-A-698 384, 764 362 oder 736 139 beschriebenen Weise oder gemäß der DE-B-1 795 797 erhalten werden.

Zu den Verfahren b) und c) des Anspruchs 9

Das Verfahren b) wird zweckmäßig bei Temperaturen zwischen 80 bis 250 °C ausgeführt, wobei falls Z eine Hydroxygruppe bedeutet, gegebenenfalls auch höhere Temperaturen bis zu 400 °C erforderlich sind. Der Verfahrensweg c) wird zweckmäßig bei Temperaturen zwischen 20 bis 150 °C durchgeführt.

Als Lösungsmittel für die Verfahren b) und c) kommen beispielsweise in Frage : Wasser, Alkohole, Benzol, Toluol, Dioxan, Dimethylformamid, N-Methylpyrrolidon, Dimethylsulfoxyd, Sulfolan, Tetramethylharnstoff und so weiter.

Unter den Kondensationsmitteln, die für das Verfahren b) falls Z oder Y ein Halogenatom bedeutet, in Betracht kommen, sind in erster Linie beispielsweise Natriumacetat, Natriumamid, Alkalicarbonate und tertiäre Amine zu nennen. Zinkchlorid, Phosphoroxychlorid, p-Toluolsulfonsäure, Jod und dergleichen können beispielsweise als Kondensationsmittel dienen, falls Z eine Aminogruppe bedeutet. Zinkchlorid, Calciumchlorid und Triäthylphosphat können beispielsweise Verwendung finden, falls Z oder Y eine Hydroxylgruppe oder die Gruppe —OW bedeuten. Die gegebenenfalls sich anschließende Acylierung der 3-ständigen Aminogruppe (Einführung von R′CO—) kann nach den bekannten Methoden mit den entsprechenden Estern durchgeführt werden (Siehe hierzu bei Verfahren a) von Anspruch 9).

Ausgangsstoffe der Formel III′, worin V die Gruppe R′CO— und Y die Aminogruppe ist, können aus 2,3,6-Triaminopyridin durch Einführung der R′CO-Gruppe erhalten werden (siehe hierzu bei Verfahren a) von Anspruch 9. Ausgangsstoffe der Formel III′, worin Y Halogen ist, können durch Reduktion von 2-Amino-3-nitro-6-chlor-pyridin und anschließender Einführung der Gruppe R′CO— erhalten werden. Zur Herstellung von entsprechenden Ausgangsverbindungen die in 6-Stellung ein Bromatom haben, wird

beispielsweise das 2-Amino-3-nitro-6-chlor-pyridin mit einer gesättigten, wässrig-alkoholischen Ammoniaklösung im Autoklav bei 100 bis 120 °C einige Stunden (2 bis 4) erhitzt und das dabei gebildete 6-Aminopyridin-derivat dann in bekannter Weise diazotiert und nach den Bedingungen der Sandmeyerreaktion beziehungsweise modifizierten Sandmeyerreaktion in Gegenwart von Bromidionen und/oder entsprechenden Kupfer-(I)-Salzen (CuBr, CuCl) unter Erwärmen umgesetzt. Als Lösungsmittel eignen sich hierfür Wasser-Alkohol-Gemische, oder Gemische von Wasser, Dimethylformamid und Dimethylsulfoxyd.

Ausgangsstoffe der Formel III', worin Y eine Hydroxygruppe, eine niedere Alkoxygruppe oder eine Phenoxygruppe ist, können beispielsweise wie folgt erhalten werden : 2,6-Dichlor-3-nitro-pyridin wird mit einem Äquivalent eines niederen Alkoholats oder Alkaliphenolat oder einem Äquivalent Alkali gegebenenfalls in Gegenwart eines tertiären Amins in einem polaren Lösungsmittel (niedere Alkohole, Tetrahydrofuran) bei Temperaturen zwischen — 50 bis + 100 °C, vorzugsweise zwischen — 50 und + 10 °C umgesetzt und in der erhaltenen Verbindung die Nitrogruppe durch Hydrierung in Gegenwart von Raney-Nickel oder sonstigen Edelmetallkatalysatoren wie Palladium oder Platin, beispielsweise bei Temperaturen zwischen 20 und 150 °C in einem Lösungsmittel wie Alkoholen, Dioxan, Tetrahydrofuran zur Aminogruppe reduziert. In den so erhaltenen Verbindungen kann dann gegebenenfalls die Gruppe R'CO— durch Umsetzung mit dem entsprechenden Säurehalogenid eingeführt werden (siehe bei Verfahren a) von Anspruch 9).

Beispiel 1

2-Amino-3-carbethoxyamino-6-(α-methyl-4-fluor-benzylamino)-pyridin

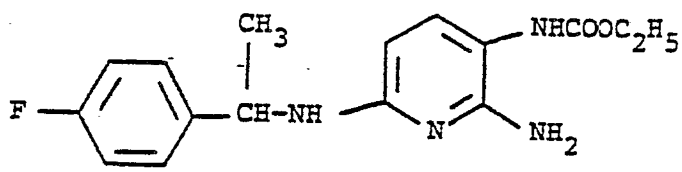

35 g 2-Amino-3-nitro-6-(α-methyl-4-fluorbenzylamino)-pyridin werden mit 10 g Ra-Nickel und 30 g Magnesiumsulfat in 500 ml Dioxan im Autoklaven bei 55 °C hydriert. Der Katalysator wird sodann unter Stickstoff abgesaugt, mit wenig Dioxan nachgewaschen und das Filtrat mit 13,8 ml Chlorameisensäureethylester versetzt. Das Reaktionsprodukt wird mit Ether und Benzin als Öl aus der Lösung gefällt. Die ölige Verbindung nimmt man in Wasser auf, stellt mit 2N-Natronlauge alkalisch und extrahiert die Base mit Ether. Die etherische Phase wird mit isopropanolischer Salzsäure versetzt, wobei ein sirupähnlicher Niederschlag ausfällt, der nach 2 Stunden kristallisiert. Die Kristalle des Hydrochlorids werden abgesaugt, gut mit einem Aceton/Ether- Gemisch nachgewaschen und anschließend getrocknet.

Ausbeute : 35 g

F. des Hydrochlorids 160 °C.

Analog Beispiel 1 werden die in Tabelle 3 aufgeführten Nitro-Verbindungen der Formel

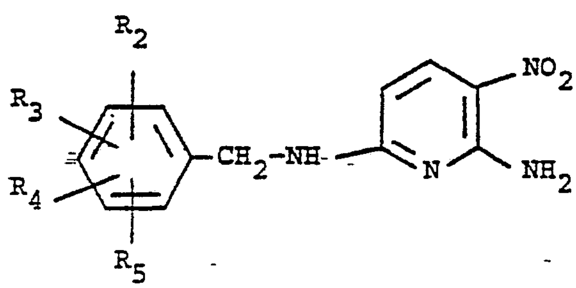

(II)

in Gegenwart von Raney-Nickel und Magnesiumsulfat unter einem Wasserstoffdruck von 40 bar (falls nichts anderes angegeben ist) hydriert und anschließend mit Chlorameisensäureethylester zu den Endverbindungen

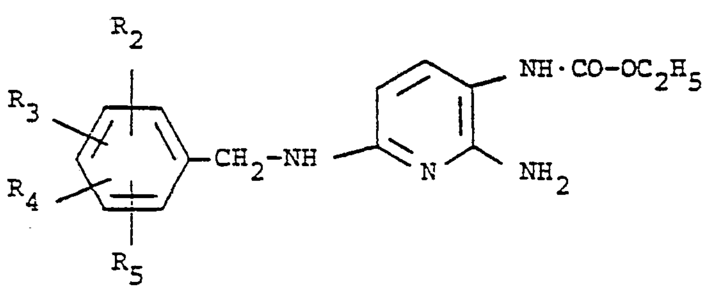

umgesetzt (Beispiele 2-7). Bei Beispiel 8 wird anstelle von Chlorameisensäureethylester mit Acetylchlorid acyliert ; man erhält daher als Endprodukt das 2-Amino-3-acetamino-6-(4-fluor-benzylamino)-pyridin. Bei Beispiel 9 wird nach der Hydrierung mit Chlorameisensäurephenylethylester acyliert ; man erhält hier das 2-Amino-3-phenylethoxycarbonylamino-6-benzylamino-pyridin.

In Spalte 9 von Tabelle 3 ist die Aufarbeitung nach dem Zusatz des Acylierungsmittels angegeben. Falls in dieser Spalte 9 nichts angegeben ist, bedeutet dies, daß das Endprodukt nach Zusatz des Chlorameisensäureethylesters beziehungsweise des Chlorameisensäurephenylethylesters oder des Acetylchlorids aus der Reaktionsmischung in Form des Hydrochlorids auskristallisiert (sofort oder im Verlauf von 1-12 Stunden). In diesen Fällen wird das Endprodukt abgesaugt, gewaschen, im Vakuum getrocknet und gegebenenfalls umkristallisiert. Falls das Endprodukt umkristallisiert wurde, ist das verwendete Lösungsmittel in Spalte 7 der Tabelle 3 angegeben.

(Siehe Tabelle 3 Seite 14 ff.)

Beispiel 10

2-Amino-3-carbethoxyamino-6-(2,4,6-trimethylbenzylamino)-pyridin-(1/3 Citrat)

40 g 2-Amino-3-carbethoxyamino-6-(2,4,6-trimethylbenzylamino)-pyridin-Hydrochlorid werden in 200 ml Methanol gelöst und mit 25 %igem Ammoniak versetzt, wobei die Base auskristallisiert. Die Base saugt man ab und löst sie in Dioxan/Methanol (2 : 1) in der Wärme. Die warme Lösung wird mit einer Lösung von Zitronensäure in Methanol auf pH 3 angesäuert, wobei unter Rühren das « Citrat » beim Abkühlen ausfällt. Die Verbindung kristallisiert mit 0,33 Mol Zitronensäure und wird aus wenig Isopropanol umkristallisiert.

Ausbeute : 33 g

F. 131-137 °C

Beispiel 11

2-Amino-3-phenoxycarbonylamino-6-(4-fluor-benzylamino)-pyridin

24,3 g 2-Amino-3-nitro-6-(4-fluor-benzylamino)-pyridin werden mit 18 g Raney-Nickel und 18 g Magnesiumsulfat in 250 ml Dioxan bei 65 °C unter einem Druck von 7,5 bar hydriert. Anschließend wird unter Stickstoff vom Katalysator abgesaugt, mit wenig Dioxan nachgewaschen und das Filtrat unter Stickstoff mit 17,1 g Chlorameisensäurephenylester versetzt. Das Reaktionsprodukt kristallisiert nach kurzer Zeit aus und wird abgesaugt. Die Verbindung wird aus Methanol unter Kohlezusatz umkristallisiert und im Vakuum getrocknet.

Ausbeute : 25 g

F. des Hydrochlorids : 210-211 °C.

Beispiele für galenische Zubereitungen

Tabletten :

10 kg 2-Amino-3-carbethoxyamino-6-(4-fluor-benzylamino)-pyridin-maleat werden mit 2,5 kg Calciumhydrogenphosphat und 2,5 kg Maisstärke gemischt und die Mischung mit einer Lösung von 1 kg Polyvinylpyrrolidon in 4 kg demineralisiertem Wasser in bekannter Weise granuliert. Nach Zumischen von 1,3 kg Maisstärke, 2 kg mikrokristalliner Cellulose, 0,6 kg Magnesiumstearat und 0,1 kg hochdispersem Siliciumdioxid werden Tabletten mit einem Gewicht von 200 mg, einem Durchmesser von 9 mm und einem Wölbungsradius von 10 mm mit Bruchkerbe gepresst. Die Bruchfestigkeit der Tabletten beträgt 80-100 N (Schleuniger-Bruchfestigtester). Die Zerfallzeit nach DAB 8 beträgt 5 Minuten.

Jede Tablette enthält 100 mg Wirkstoff.

Kapseln :

Analog der zuvor beschriebenen Herstellungsweise für Tabletten wird eine Kapselfüllung hergestellt, die in Hartgelatine-Kapseln der passenden Grösse abgefüllt wird. Füllmenge pro Kapsel : 200 mg.

Eine Kapsel enthält 100 mg Wirkstoff.

Tabelle 3

| Beispiel-Nr. | 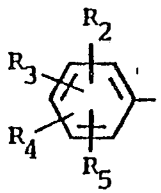 | Nitro-Verbindung II g | Ra-Nickel/$MgSO_4$ in g | Temperatur der Hydrierung | Chlorameisensäureethylester ml | F. des Endproduktes C° als Hydrochlorid | Ausbeute g | Aufarbeitung |
|---|---|---|---|---|---|---|---|---|
| 2 | 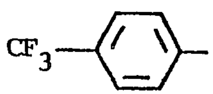 | 8,5 | 6/20 | 40° C | 3 | 200 | 9 | |
| 3 | 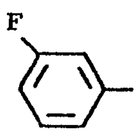 | 25 | 5/20 | 50° C | 9,3 | 205 | 26 | |
| 4 | 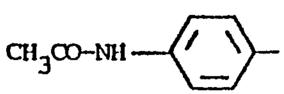 | 12 | 9/30 | 90° C | 4,5 | 211-212 | 8 | das Reaktionsprodukt fällt ölig aus und kristalliert nach kurzem Stehen; Umkristallisation zuerst aus Wasser, dann aus Isopropanol |

Tabelle 3 (Fortsetzung)

| Beispiel-Nr. | 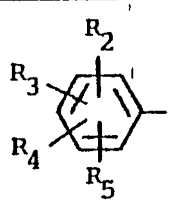 | Nitro-Verbindung II g | Ra-Nickel/ $MgSO_4$ in g | Temperatur der Hydrierung | Chlorameisensäureethylester ml | F. des Endproduktes C° als Hydrochlorid | Ausbeute g | Aufarbeitung |
|---|---|---|---|---|---|---|---|---|
| 5 | 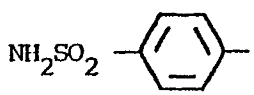 | 65 | 30/60 | 80° C | 22,2 | 168–171 | 45 | wie bei Beispiel 4, zusätzlich Etherzusatz, Umkristallisation aus Methanol |
| 6 | 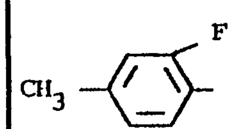 | 42,5 | 15/30 | 40° C | 16,8 | 212–213; Methanol | 26 | Absaugen nach 12 Stunden |
| 7 | 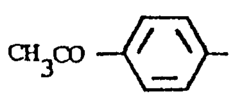 1) | 44,5 | 30/60 | 60° C | 16,5 | 203–206 | 10 | Animpfen und Absaugen nach Stehen über Nacht |

1) Als Ausgangsverbindung II wird die geschützte Keto-Verbindung (in Form des Ethylenacetats) eingesetzt.

Tabelle 3 (Fortsetzung)

| Beispiel-Nr. | 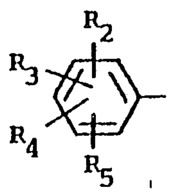 | Nitro-Verbindung II g | Ra-Nickel/ $MgSO_4$ in g | Temperatur der Hydrierung | Chlorameisensäureethylester ml | F. des Endproduktes C° als Hydrochlorid | Ausbeute g | Aufarbeitung |
|---|---|---|---|---|---|---|---|---|
| 8 | F 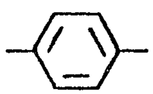 | 46 | 35/35 | 65° C; 64 bar | 15,7 Acetylchlorid | 247-249; Methanol | 6 | |
| 9 | 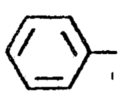 | 99 | 30/40 | 50° C; 20 bar | 80 Chlorameisensäurephenylethylester | 199; Methanol | | |

**Patentansprüche** (für die Vertragsstaaten : BE, CH, DE, FR, GB, IT, LI, LU, NL, SE)

1. Verbindungen der Formel

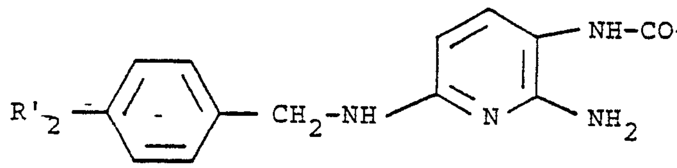

I′

worin R′ eine $C_1$-$C_4$-Alkylgruppe, eine $C_1$-$C_4$-Alkoxygruppe, eine Phenoxygruppe oder eine Phenyl-$C_1$-$C_2$-alkoxygruppe bedeutet und $R'_2$ eine Trifluormethylgruppe, eine $C_1$-$C_4$-Alkylcarbonylgruppe, eine $C_1$-$C_4$-Alkylcarbonylaminogruppe oder eine Aminosulfonylgruppe ($NH_2SO_2$—) darstellt und wobei $R'_2$ auch Fluor oder Wasserstoff sein kann, falls R′ eine $C_1$-$C_4$-Alkylgruppe, eine Phenoxygruppe oder eine Phenyl-$C_1$-$C_2$-alkoxygruppe ist.

2. Verbindungen nach Anspruch 1, worin R′ eine $C_1$-$C_4$-Alkylgruppe, eine $C_1$-$C_4$-Alkoxygruppe, eine Phenoxygruppe oder eine Phenyl-$C_1$-$C_2$-alkoxygruppe bedeutet und $R'_2$ eine Trifluormethylgruppe, eine Acetylgruppe, eine Acetylaminogruppe oder eine Aminosulfonylgruppe darstellt und wobei $R'_2$ auch Fluor oder Wasserstoff sein kann, falls R′ eine $C_1$-$C_4$-Alkylgruppe, eine Phenoxygruppe oder eine Phenyl-$C_1$-$C_2$-alkoxygruppe ist.

3. Verbindungen nach Anspruch 1, worin $R'_2$ eine Trifluormethylgruppe ist.

4. Verbindungen nach Anspruch 1, worin R′ eine $C_1$-$C_4$-Alkoxygruppe oder eine Phenoxygruppe und $R'_2$ eine Trifluormethylgruppe ist.

5. Verbindungen nach Anspruch 1, worin R′ eine $C_1$-$C_4$-Alkylgruppe, eine Phenoxygruppe oder eine Phenyl-$C_1$-$C_2$-alkoxygruppe ist und $R'_2$ Wasserstoff oder Fluor ist.

6. Antiepileptisch wirkendes Arzneimittel, enthaltend mindestens eine Verbindung der Formel I′

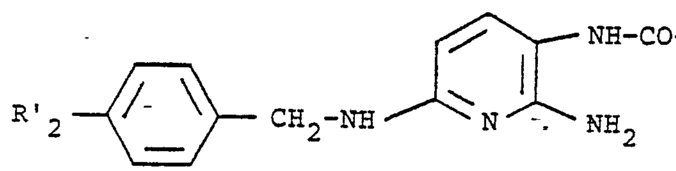

I′

worin R′ eine $C_1$-$C_4$-Alkylgruppe, eine $C_1$-$C_4$-Alkoxygruppe, eine Phenoxygruppe oder eine Phenyl-$C_1$-$C_2$-alkoxygruppe bedeutet und $R'_2$ eine Trifluormethylgruppe, eine $C_1$-$C_4$-Alkylcarbonylgruppe, eine $C_1$-$C_4$-Alkylcarbonylaminogruppe oder eine Aminosulfonylgruppe ($NH_2SO_2$—) darstellt und wobei $R'_2$ auch Fluor oder Wasserstoff sein kann, falls R′ eine $C_1$-$C_4$-Alkylgruppe, eine Phenoxygruppe oder eine Phenyl-$C_1$-$C_2$-alkoxygruppe ist oder deren Säureadditionssalzen.

7. Antiepileptisch wirkendes Arzneimittel nach Anspruch 6, dadurch gekennzeichnet, daß es außerdem übliche inerte Träger- und/oder Verdünnungsstoffe enthält.

8. Verfahren zur Herstellung eines antiepileptisch wirkenden Arzneimittels, dadurch gekennzeichnet, daß mindestens eine Verbindung nach einem oder mehreren der Ansprüche 1-5 mit gebräuchlichen pharmazeutischen Trägerstoffen beziehungsweise Verdünnungsmitteln zu einem Arzneimittel zur Behandlung von Epilepsie verarbeitet wird.

9. Verfahren zur Herstellung von Verbindungen gemäß Anspruch 1, dadurch gekennzeichnet, daß man

a) in einer Verbindung der Formel

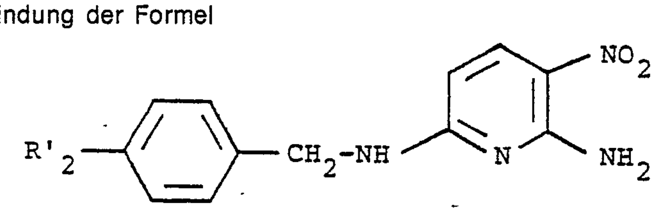

II′

worin der Rest $R'_2$ die angegebenen Bedeutungen hat, die Nitrogruppe zur Aminogruppe reduziert und in die so erhaltene 3-ständige Aminogruppe durch Acylierung den Rest R′CO— einführt, wobei R′ die angegebenen Bedeutungen hat oder

b) eine Verbindung der allgemeinen Formel

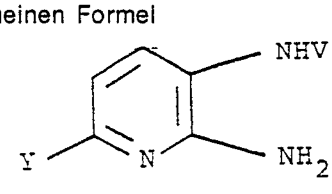

III′

mit einer Verbindung der allgemeinen Formel

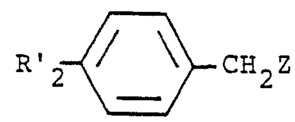

IV'

umsetzt, wobei V der Formel III' Wasserstoff oder die Gruppe —COR' ist und Y eine Aminogruppe bedeutet, falls Z ein Halogenatom, eine Aminogruppe oder die Gruppe

—OW

darstellt und wo W ein Wasserstoffatom, eine niedrigmolekulare Alkylgruppe oder eine gegebenenfalls durch Halogenatome, Nitrogruppen oder Methylgruppen substituierte Phenylgruppe ist oder Z auch eine Aminogruppe sein kann, falls Y Halogen, eine Hydroxygruppe oder die Gruppe OW ist, mit oder ohne Lösungsmittel, vorzugsweise in Gegenwart eines Kondensationsmittels bei erhöhter Temperatur umsetzt oder

c) eine Verbindung der allgemeinen Formel III', worin Y die Aminogruppe ist und V die angegebenen Bedeutungen hat, mit einer Verbindung der allgemeinen Formel

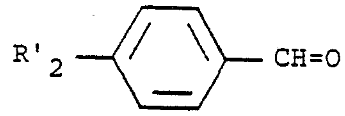

V'

unter gleichzeitiger Reduktion kondensiert und in die nach den Verfahren b) bis c) erhaltenen Verbindungen, die in 3-Stellung des Pyridinringes eine freie Aminogruppe haben, in diese 3-ständige Aminogruppe durch Acylierung den Rest R'CO— einführt und gegebenenfalls die nach den Verfahren a) bis c) erhaltenen Verbindungen in ihre Säureadditionssalze überführt.

10. Verwendung von Verbindungen der Formel I

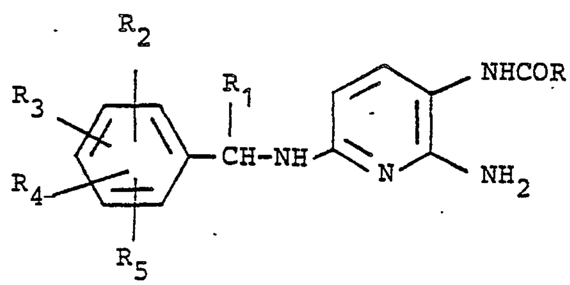

(I)

worin R eine $C_1$-$C_4$-Alkylgruppe, eine $C_1$-$C_4$-Alkoxygruppe, eine Phenoxygruppe oder eine Phenyl-$C_1$-$C_2$-alkoxygruppe, $R_1$ Wasserstoff oder eine $C_1$-$C_4$-Alkylgruppe und $R_5$ Wasserstoff oder eine $C_1$-$C_4$-Alkylgruppe bedeutet und die Reste $R_2$, $R_3$ und $R_4$ gleich oder verschieden sind und Wasserstoff, Trifluormethyl, Halogenatome, $C_1$-$C_4$-Alkylgruppen, $C_1$-$C_4$-Alkylcarbonylgruppen, die Aminosulfonylgruppe ($NH_2$—$SO_2$—) oder eine $C_1$-$C_4$-Alkylcarbonylaminogruppe bedeuten und deren Säureadditionssalzen für die Herstellung eines Arzneimittels zur Behandlung von Epilepsie.

11. Verwendung von Verbindungen der Formel I

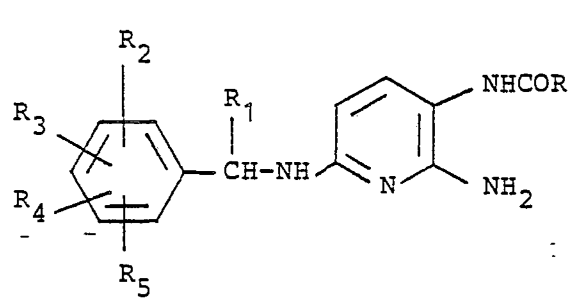

(I)

worin R eine $C_1$-$C_4$-Alkylgruppe, eine $C_1$-$C_4$-Alkoxygruppe, eine Phenoxygruppe oder eine Phenyl-$C_1$-$C_2$-alkoxygruppe, $R_1$ Wasserstoff oder eine $C_1$-$C_4$-Alkylgruppe und $R_2$ Wasserstoff, Trifluormethyl, Halogen, eine $C_1$-$C_4$-Alkylcarbonylgruppe, eine $C_1$-$C_4$-Alkylcarbonylaminogruppe oder eine Aminosulfonylgruppe ist, die Reste $R_3$ und $R_4$ gleich oder verschieden sind und Wasserstoff, Halogenatome oder $C_1$-$C_4$-Alkylgruppen darstellen und $R_5$ Wasserstoff oder eine $C_1$-$C_4$-Alkylgruppe ist und deren Säureadditionssalzen für die Herstellung eines Arzneimittels zur Behandlung von Epilepsie.

12. Verwendung von Verbindungen der Formel I

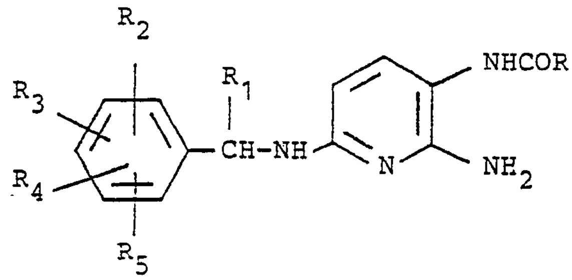

(I)

worin R eine $C_1$-$C_4$-Alkylgruppe, eine $C_1$-$C_4$-Alkoxygruppe, eine Phenoxygruppe oder eine Phenyl-$C_1$-$C_2$-alkoxygruppe, $R_1$ Wasserstoff oder eine $C_1$-$C_4$-Alkylgruppe, $R_2$ Wasserstoff, Trifluormethyl, Halogen, eine $C_1$-$C_4$-Alkylcarbonylgruppe, eine $C_1$-$C_4$-Alkylcarbonylaminogruppe oder eine Aminosulfonylgruppe ist und die Reste $R_3$, $R_4$ und $R_5$ Wasserstoff sind und deren Säureadditionssalzen für die Herstellung eines Arzneimittels zur Behandlung von Epilepsie.

13. Verwendung von Verbindungen der Formel I

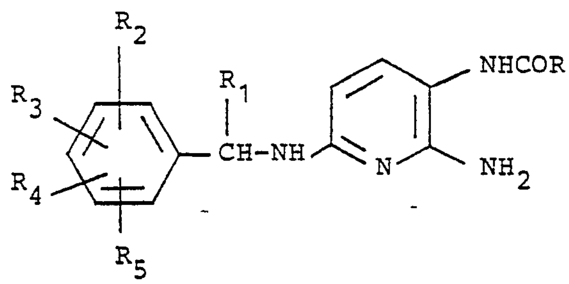

(I)

worin R eine $C_1$-$C_4$-Alkoxygruppe, $R_2$ Wasserstoff, Trifluormethyl, Halogen, eine $C_1$-$C_4$-Alkylcarbonylgruppe, eine $C_1$-$C_4$-Alkylcarbonylaminogruppe oder eine Aminosulfonylgruppe ist und die Reste $R_1$, $R_3$, $R_4$ und $R_5$ Wasserstoff sind und deren Säureadditionssalzen für die Herstellung eines Arzneimittels zur Behandlung von Epilepsie.

14. Verwendung von Verbindungen der Formel I

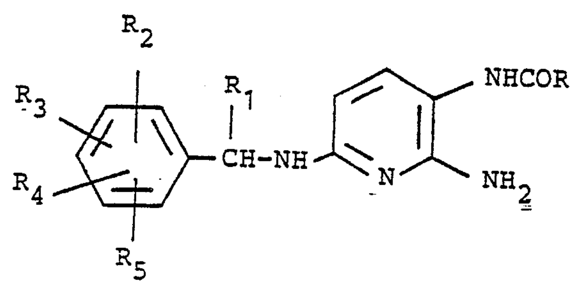

(I)

worin R eine $C_1$-$C_4$-Alkylgruppe, eine Phenoxygruppe oder eine Phenyl-$C_1$-$C_2$-alkoxygruppe, $R_2$ Wasserstoff, Trifluormethyl, Halogen, eine $C_1$-$C_4$-Alkylcarbonylgruppe, eine $C_1$-$C_4$-Alkylcarbonylaminogruppe oder eine Aminosulfonylgruppe ist und die Reste $R_1$, $R_3$, $R_4$ und $R_5$ Wasserstoff sind und deren Säureadditionssalzen für die Herstellung eines Arzneimittels zur Behandlung von Epilepsie.

15. Verwendung von Verbindungen nach einem oder mehreren der Ansprüche 1-5 für die Herstellung eines Arzneimittels zur Behandlung von Epilepsie.

**Patentansprüche** (für den Vertragsstaat AT)

1. Verfahren zur Herstellung von Verbindungen der Formel

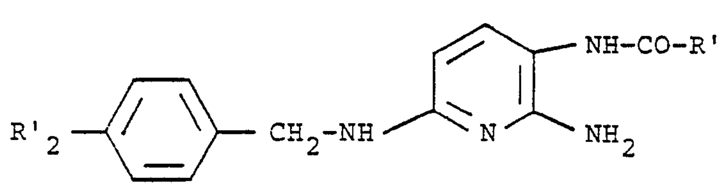

I'

worin R′ eine $C_1$-$C_4$-Alkylgruppe, eine $C_1$-$C_4$-Alkoxygruppe, eine Phenoxygruppe oder eine Phenyl-$C_1$-$C_2$-alkoxygruppe bedeutet und $R'_2$ eine Trifluormethylgruppe, eine $C_1$-$C_4$-Alkylcarbonylgruppe, eine $C_1$-$C_4$-Alkylcarbonylaminogruppe oder eine Aminosulfonylgruppe ($NH_2SO_2$—) darstellt und wobei $R'_2$ auch Fluor oder Wasserstoff sein kann, falls R′ eine $C_1$-$C_4$-Alkylgruppe, eine Phenoxygruppe oder eine Phenyl-$C_1$-$C_2$-alkoxygruppe ist, dadurch gekennzeichnet, daß man

a) in einer Verbindung der Formel

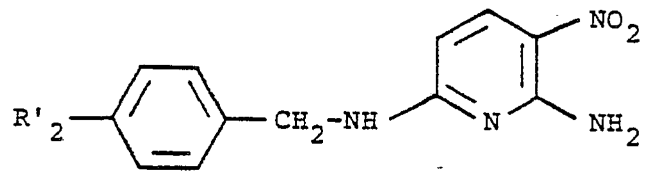

II′

worin der Rest $R'_2$ die angegebenen Bedeutungen hat, die Nitrogruppe zur Aminogruppe reduziert und in die so erhaltene 3-ständige Aminogruppe durch Acylierung den Rest R′CO— einführt, wobei R′ die angegebenen Bedeutungen hat oder

b) eine Verbindung der allgemeinen Formel

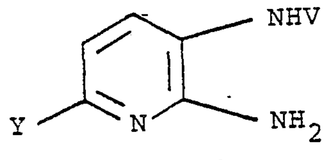

III′

mit einer Verbindung der allgemeinen Formel

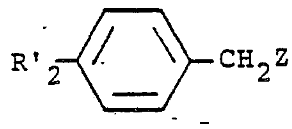

IV′

umsetzt, wobei V der Formel III′ Wasserstoff oder die Gruppe —COR′ ist und Y eine Aminogruppe bedeutet, falls Z ein Halogenatom, eine Aminogruppe oder die Gruppe

—OW

darstellt und wo W ein Wasserstoffatom, eine niedrigmolekulare Alkylgruppe oder eine gegebenenfalls durch Halogenatome, Nitrogruppen oder Methylgruppen substituierte Phenylgruppe ist oder Z auch eine Aminogruppe sein kann, fall Y Halogen, eine Hydroxygruppe oder die Gruppe OW ist, mit oder ohne Lösungsmittel, vorzugsweise in Gegenwart eines Kondensationsmittels bei erhöhter Temperatur umsetzt oder

c) eine Verbindung der allgemeinen Formel III′, worin Y die Aminogruppe ist und V die angegebenen Bedeutungen hat, mit einer Verbindung der allgemeinen Formel

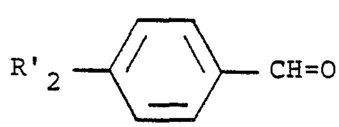

V′

unter gleichzeitiger Reduktion kondensiert und in die nach den Verfahren b) bis c) erhaltenen Verbindungen, die in 3-Stellung des Pyridinringes eine freie Aminogruppe haben, in diese 3-ständige Aminogruppe durch Acylierung den Rest R′CO— einführt und gegebenenfalls die nach den Verfahren a) bis c) erhaltenen Verbindungen in ihre Säureadditionssalze überführt.

2. Verfahren nach Anspruch 1, dadurch gekennzeichnet, daß Verbindungen der Formel I hergestellt werden, worin R′ eine $C_1$-$C_4$-Alkylgruppe, eine $C_1$-$C_2$-Alkoxygruppe, eine Phenoxygruppe oder eine Phenyl-$C_1$-$C_2$-alkoxygruppe bedeutet und $R'_2$ eine Trifluormethylgruppe, eine Acetylgruppe, eine Acetylaminogruppe oder eine Aminosulfonylgruppe darstellt und wobei $R'_2$ auch Fluor oder Wasserstoff sein kann, falls R′ eine $C_1$-$C_4$-Alkylgruppe, eine Phenoxygruppe oder eine Phenyl-$C_1$-$C_2$-alkoxygruppe ist.

3. Verfahren nach Anspruch 1, dadurch gekennzeichnet, daß Verbindungen der Formel I hergestellt werden, worin $R'_2$ eine Trifluormethylgruppe ist.

4. Verfahren nach Anspruch 1, dadurch gekennzeichnet, daß Verbindungen der Formel I hergestellt

werden, worin R' eine $C_1$-$C_4$-Alkoxygruppe oder eine Phenoxygruppe und $R'_2$ eine Trifluormethylgruppe ist.

5. Verfahren nach Anspruch 1, dadurch gekennzeichnet, daß Verbindungen der Formel I hergestellt werden, worin R' eine $C_1$-$C_4$-Alkylgruppe, eine Phenoxygruppe oder eine Phenyl-$C_1$-$C_2$-alkoxygruppe ist und $R'_2$ Wasserstoff oder Fluor ist.

6. Verwendung von Verbindungen der Formel I

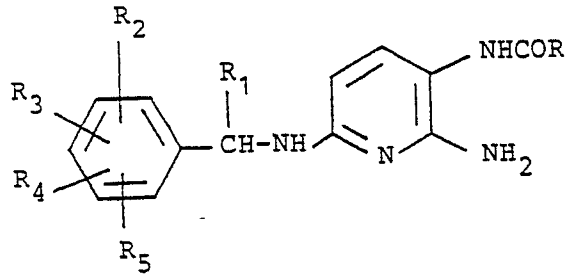

(I)

worin R eine $C_1$-$C_4$-Alkylgruppe, eine $C_1$-$C_4$-Alkoxygruppe, eine Phenoxygruppe oder eine Phenyl-$C_1$-$C_2$-alkoxygruppe, $R_1$ Wasserstoff oder eine $C_1$-$C_4$-Alkylgruppe und $R_5$ Wasserstoff oder eine $C_1$-$C_4$-Alkylgruppe bedeutet und die Reste $R_2$, $R_3$ und $R_4$ gleich oder verschieden sind und Wasserstoff, Trifluormethyl, Halogenatome, $C_1$-$C_4$-Alkylgruppen, $C_1$-$C_4$-Alkylcarbonylgruppen, die Aminosulfonylgruppe ($NH_2$—$SO_2$—) oder eine $C_1$-$C_4$-Alkylcarbonylaminogruppe bedeuten und deren Säureadditionssalzen für die Herstellung eines Arzneimittels zur Behandlung von Epilepsie.

7. Verwendung von Verbindungen der Formel I

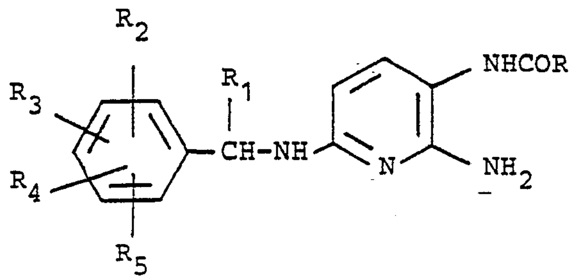

(I)

worin R eine $C_1$-$C_4$-Alkylgruppe, eine $C_1$-$C_4$-Alkoxygruppe, eine Phenoxygruppe oder eine Phenyl-$C_1$-$C_2$-alkoxygruppe, $R_1$ Wasserstoff oder eine $C_1$-$C_4$-Alkylgruppe und $R_2$ Wasserstoff, Trifluormethyl, Halogen, eine $C_1$-$C_2$-Alkylcarbonylgruppe, eine $C_1$-$C_4$-Alkylcarbonylaminogruppe oder eine Aminosulfonylgruppe ist, die Reste $R_3$ und $R_4$ gleich oder verschieden sind und Wasserstoff, Halogenatome oder $C_1$-$C_4$-Alkylgruppen darstellen und $R_5$ Wasserstoff oder eine $C_1$-$C_4$-Alkylgruppe ist und deren Säureadditionssalzen für die Herstellung eines Arzneimittels zur Behandlung von Epilepsie.

8. Verwendung von Verbindungen der Formel I

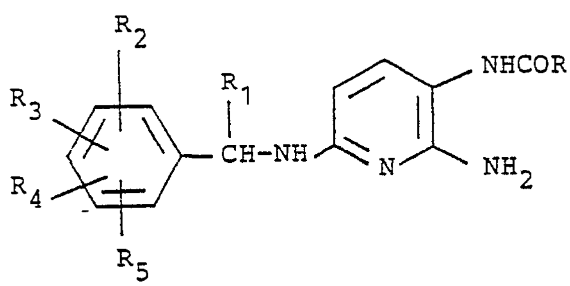

(I)

worin R eine $C_1$-$C_4$-Alkylgruppe, eine $C_1$-$C_4$-Alkoxygruppe, eine Phenoxygruppe oder eine Phenyl-$C_1$-$C_2$-alkoxygruppe, $R_1$ Wasserstoff oder eine $C_1$-$C_4$-Alkylgruppe, $R_2$ Wasserstoff, Trifluormethyl, Halogen, eine $C_1$-$C_4$-Alkylcarbonylgruppe, eine $C_1$-$C_4$-Alkylcarbonylaminogruppe oder eine Aminosulfonylgruppe ist und die Reste $R_3$, $R_4$ und $R_5$ Wasserstoff sind und deren Säureadditionssalzen für die Herstellung eines Arzneimittels zur Behandlung von Epilepsie.

9. Verwendung von Verbindungen der Formel I

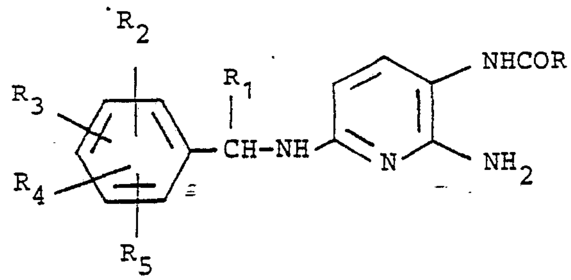

(I)

worin R eine $C_1$-$C_4$-Alkoxygruppe, $R_2$ Wasserstoff, Trifluormethyl, Halogen, eine $C_1$-$C_4$-Alkylcarbonylgruppe, eine $C_1$-$C_4$-Alkylcarbonylaminogruppe oder eine Aminosulfonylgruppe ist und die Reste $R_1$, $R_3$, $R_4$ und $R_5$ Wasserstoff sind und deren Säureadditionssalzen für die Herstellung eines Arzneimittels zur Behandlung von Epilepsie.

10. Verwendung von Verbindungen der Formel I

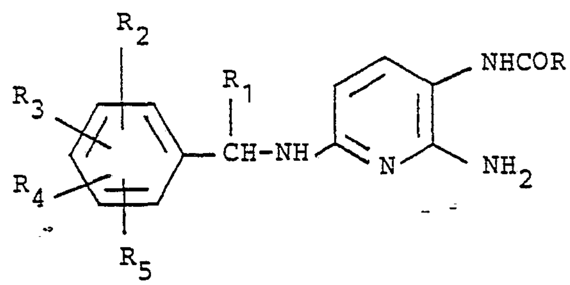

(I)

worin R eine $C_1$-$C_4$-Alkylgruppe, eine Phenoxygruppe oder eine Phenyl-$C_1$-$C_2$-alkoxygruppe, $R_2$ Wasserstoff, Trifluormethyl, Halogen, eine $C_1$-$C_4$-Alkylcarbonylgruppe, eine $C_1$-$C_4$-Alkylcarbonylaminogruppe oder eine Aminosulfonylgruppe ist und die Reste $R_1$, $R_3$, $R_4$ und $R_5$ Wasserstoff sind und deren Säureadditionssalzen für die Herstellung eines Arzneimittels zur Behandlung von Epilepsie.

11. Verwendung von Verbindungen nach einem oder mehreren der Ansprüche 1-5 für die Herstellung eines Arzneimittels zur Behandlung von Epilepsie.

**Claims** (for the contracting States : BE, CH, DE, FR, GB, LI, LU, NL, SE)

1. Compounds corresponding to the formula

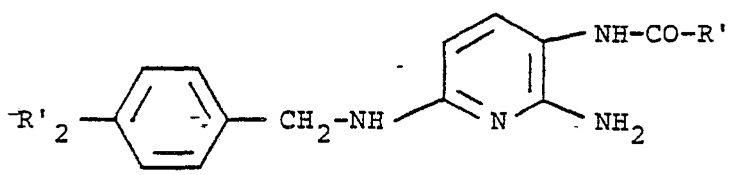

I′

wherein R′ represents a $C_1$-$C_4$-alkyl group, a $C_1$-$C_4$-alkoxy group, a phenoxy group or a phenyl-$C_1$-$C_2$-alkoxy group and $R'_2$ represents a trifluoromethyl group, a $C_1$-$C_4$-alkylcarbonyl group, a $C_1$-$C_4$-alkylcarbonylamino group or an aminosulphonyl group ($NH_2SO_2$—) and wherein $R'_2$ can also be fluorine or hydrogen if R′ is a $C_1$-$C_4$-alkyl group, a phenoxy group or a phenyl-$C_1$-$C_2$-alkoxy group.

2. Compounds according to claim 1, wherein R′ represents a $C_1$-$C_4$-alkyl group, a $C_1$-$C_4$-alkoxy group, a phenoxy group or a phenyl-$C_1$-$C_2$-alkoxy group and $R'_2$ represents a trifluoromethyl group, an acetyl group, an acetylamino group or an aminosulphonyl group and wherein $R'_2$ may also be fluorine or hydrogen, if R′ is a $C_1$-$C_4$-alkyl group, a phenoxy group or a phenyl-$C_1$-$C_2$-alkoxy group.

3. Compounds according to claim 1, wherein $R'_2$ is a trifluoromethyl group.

4. Compounds according to claim 1, wherein R′ is a $C_1$-$C_4$-alkoxy group or a phenoxy group and $R'_2$ is a trifluoromethyl group.

5. Compounds according to claim 1, wherein R′ id a $C_1$-$C_4$-alkyl group, a phenoxy group or a phenyl-$C_1$-$C_2$-alkoxy group and $R'_2$ is hydrogen or fluorine.

6. A pharmaceutical composition having an anti-epileptic effect containing at least one compound corresponding to formula I′

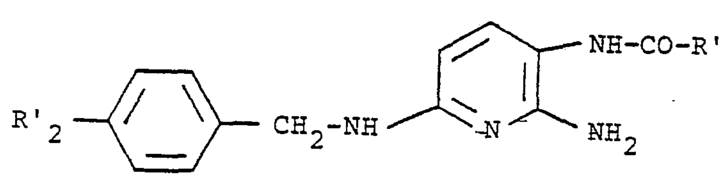

I′

wherein R' represents a $C_1$-$C_4$-alkyl group, a $C_1$-$C_4$-alkoxy group, a phenoxy group or a phenyl-$C_1$-$C_2$-alkoxy group and $R'_2$ represents a trifluoromethyl group, a $C_1$-$C_4$-alkylcarbonyl group or an aminosulphonyl group ($NH_2SO_2$—) and wherein $R'_2$ may also be fluorine or hydrogen if R' is a $C_1$-$C_4$-alkyl group, a phenoxy group or a phenyl-$C_1$-$C_2$-alkoxy group or the acid addition salts thereof.

7. A pharmaceutical composition having an anti-epileptic effect according to claim 6, characterised in that it also contains conventional inert carrier and/or diluent substances.

8. A process for the production of a pharmaceutical composition having an anti-epileptic effect, characterised in that at least one compound according, to one or more of claims 1 to 5 is processed with conventional pharmaceutical carrier substances or diluents to form a pharmaceutical composition for the treatment of epilepsy.

9. A process for the production of compounds according to claim 1, characterised in that,

a) in a compound corresponding to the formula

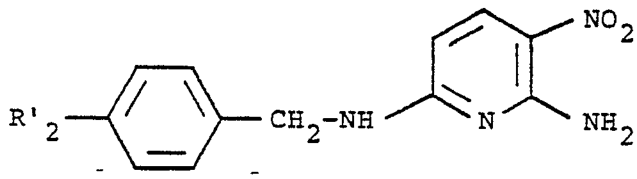

II'

wherein the radical $R'_2$ has the meanings given, the nitro group is reduced to an amino group and the radical R'CO— is introduced into the 3-position amino group thus obtained by acylation, R' having the meanings given or

b) a compound corresponding to the general formula

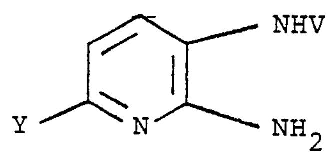

III'

is reacted with a compound corresponding to the general formula

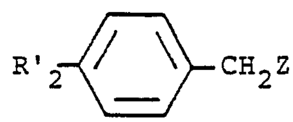

IV'

wherein V in Formula III' is hydrogen or the group —COR' and Y represents an amino group if Z is a halogen atom, an amino group or the group —OW and wherein W represents a hydrogen atom, a low molecular weight alkyl group or a phenyl group optionally substituted by halogen atoms, nitro groups or methyl groups or Z can also be an amino group if Y is halogen, a hydroxy group or the group OW, the reaction being carried out without solvent, preferably in the presence of a condensation agent at elevated temperature or

c) a compound corresponding to general formula III', wherein Y is an amino group and V has the meanings given is condensed with a compound corresponding to general formula

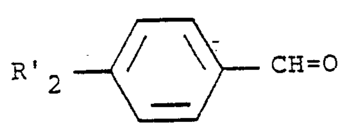

V'

with simultaneous reduction and the radical R'CO— is introduced into the compounds which are obtained according to methods b) to c) and which have a free amino group in the 3-position of the pyridine ring, by acylation of this 3-position amino group and the compounds obtained by methods a) to c) are optionally converted into the acid addition salts thereof.

10. Use of compounds corresponding to formula I

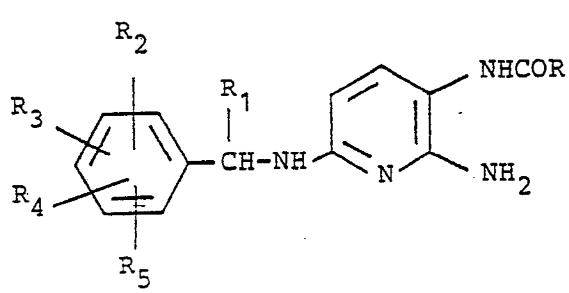

(I)

wherein R represents a $C_1$-$C_4$-alkyl group, a $C_1$-$C_4$-alkoxy group, a phenoxy group or a phenyl-$C_1$-$C_2$-alkoxy group, $R_1$ represents hydrogen or a $C_1$-$C_4$-alkyl group and $R_5$ represents hydrogen or a $C_1$-$C_4$-alkyl group and the radicals $R_2$, $R_3$ and $R_4$ are the same or different and represent hydrogen, trifluoromethyl, halogen atoms, $C_1$-$C_4$-alkyl groups, $C_1$-$C_4$-alkylcarbonyl groups, the aminosulphonyl group ($NH_2$—$SO_2$—) or a $C_1$-$C_4$-alkylcarbonylamino group and the acid addition salts thereof for the production of a pharmaceutical composition for the treatment of epilepsy.

11. Use of the compounds corresponding to formula I

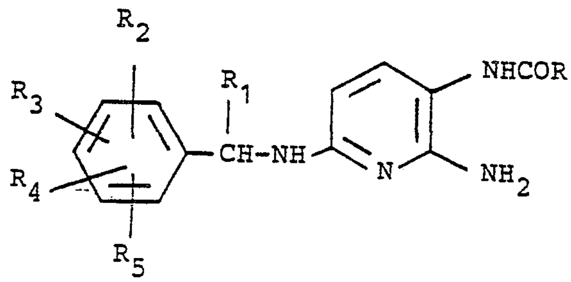

(I)

wherein R is a $C_1$-$C_4$-alkyl group, a $C_1$-$C_4$-alkoxy group, a phenoxy group or a phenyl-$C_1$-$C_2$-alkoxy group, $R_1$ is hydrogen or a $C_1$-$C_4$-alkyl group and $R_2$ is hydrogen, trifluoromethyl, halogen, a $C_1$-$C_4$-alkylcarbonyl group, a $C_1$-$C_4$-alkylcarbonylamino group or an aminosulphonyl group, the radicals $R_3$ and $R_4$ which may be the same or different represent hydrogen, halogen atoms or $C_1$-$C_4$-alkyl groups and $R_5$ is hydrogen or a $C_1$-$C_4$-alkyl group and the acid addition salts thereof for the production of a pharmaceutical composition for the treatment of epilepsy.

12. Use of the compounds corresponding to formula I

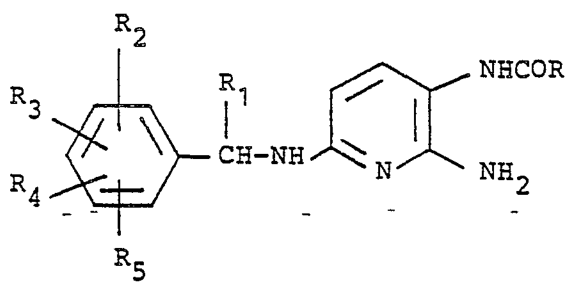

(I)

wherein R is a $C_1$-$C_4$-alkyl group, a $C_1$-$C_4$-alkoxy group, a phenoxy group or a phenyl-$C_1$-$C_2$-alkoxy group, $R_1$ is hydrogen or a $C_1$-$C_4$-alkyl group, $R_2$ is hydrogen, trifluoromethyl, halogen, a $C_1$-$C_4$-alkylcarbonyl group, a $C_1$-$C_4$-alkylcarbonylamino group or an aminosulphonyl group and the radicals $R_3$, $R_4$ and $R_5$ are hydrogen and the acid addition salts thereof for the production of a pharmaceutical composition for the treatment of epilepsy.

13. Use of the compounds corresponding to formula I

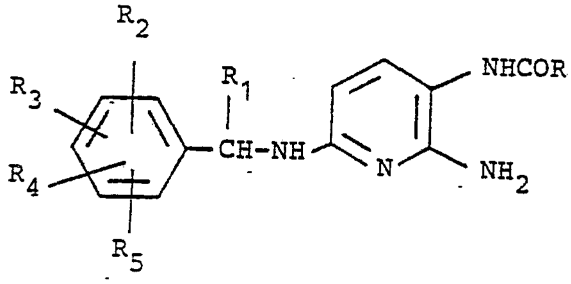

(I)

wherein R is a $C_1$-$C_4$-alkoxy group, $R_2$ is hydrogen, trifluoromethyl, halogen, a $C_1$-$C_4$-alkylcarbonyl group, a $C_1$-$C_4$-alkylcarbonylamino group or an aminosulphonyl group and the radicals $R_1$, $R_3$, $R_4$ and $R_5$ are hydrogen, and the acid addition salts thereof for the production of a pharmaceutical composition for the treatment of epilepsy.

14. Use of the compounds corresponding to formula I

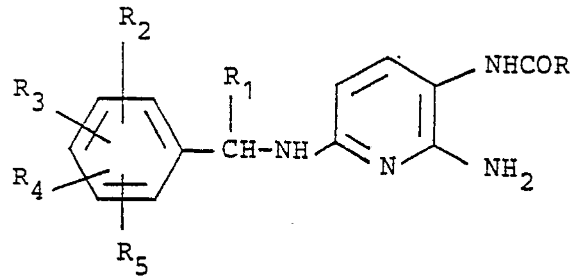

(I)

wherein R is a $C_1$-$C_4$-alkyl group, a phenoxy group or a phenyl-$C_1$-$C_2$-alkoxy group, $R_2$ is hydrogen, trifluoromethyl, halogen, a $C_1$-$C_4$-alkylcarbonyl group, a $C_1$-$C_4$-alkylcarbonylamino group or an aminosulphonyl group, and the radicals $R_1$, $R_3$, $R_4$ and $R_5$ are hydrogen, and the acid addition salts thereof for the production of a pharmaceutical composition for the treatment of epilepsy.

15. Use of compounds according to one or more of Claims 1 to 5 for the production of a pharmaceutical composition for the treatment of epilepsy.

**Claims** (for the Contracting State AT)

1. A process for the production of compounds corresponding to the formula

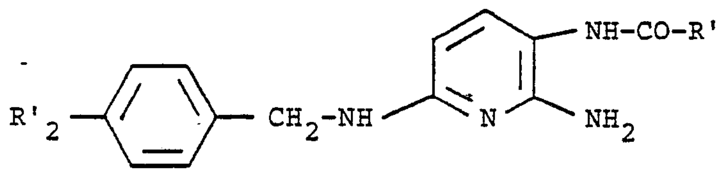

I'

wherein R' represents a $C_1$-$C_4$-alkyl group, a $C_1$-$C_4$-alkoxy group, a phenoxy group or a phenyl-$C_1$-$C_2$-alkoxy group and $R'_2$ represents a trifluoromethyl group, a $C_1$-$C_4$-alkylcarbonyl group, a $C_1$-$C_4$-alkylcarbonylamino group or an aminosulphonyl group ($NH_2SO_2$—) and wherein $R'_2$ can also be fluorine or hydrogen if R' is a $C_1$-$C_4$-alkyl group, a phenoxy group or a phenyl-$C_1$-$C_2$-alkoxy group, characterised in that

a) in a compound corresponding to the formula

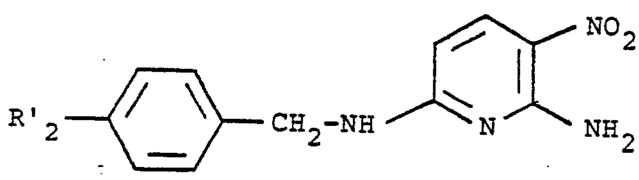

II'

wherein the radical $R'_2$ has the meanings given, the nitro group is reduced to an amino group and the radical R'CO— is introduced into the 3-position amino group thus obtained by acylation, R' having the meanings given or

b) a compound corresponding to the general formula

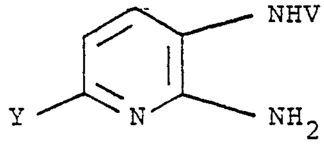

III'

is reacted with a compound corresponding to the general formula

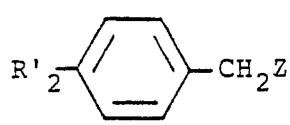

IV'

wherein V in Formula III' is hydrogen or the group —COR' and Y represents an amino group if Z is a

halogen atom, an amino group or the group —OW and wherein W represents a hydrogen atom, a low molecular weight alkyl group or a phenyl group optionally substituted by halogen atoms, nitro groups or methyl groups or Z can also be an amino group if Y is halogen, a hydroxy group or the group OW, the reaction being carried out without solvent, preferably in the presence of a condensation agent at elevated temperature or

c) a compound corresponding to general formula III', wherein Y is an amino group and V has the meanings given is condensed with a compound corresponding to general formula

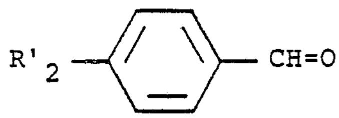

V'

with simultaneous reduction and the radical R'CO— is introduced into the compounds which are obtained according to methods b) to c) and which have a free amino group in the 3-position of the pyridine ring, by acylation of this 3-position amino group and the compounds obtained by methods a) to c) are optionally converted into the acid addition salts thereof.

2. A process according to Claim 1, characterised in that compounds corresponding to formula I are produced, wherein R' represents a $C_1$-$C_4$-alkyl group, a $C_1$-$C_4$-alkoxy group, a phenoxy group or a phenyl-$C_1$-$C_2$-alkoxy group and $R'_2$ represents a trifluoromethyl group, an acetyl group, an acetylamino group or an aminosulphonyl group and wherein $R'_2$ can also be fluorine or hydrogen if R' is a $C_1$-$C_4$-alkyl group, a phenoxy group or a phenyl-$C_1$-$C_2$-alkoxy group.

3. A process according to Claim 1, characterised in that compounds corresponding to formula I are produced, wherein $R'_2$ is a trifluoromethyl group.

4. A process according to Claim 1, characterised in that compounds corresponding to formula I are produced, wherein R' is a $C_1$-$C_4$-alkoxy group or a phenoxy group and $R'_2$ is a trifluoromethyl group.

5. A process according to Claim 1, characterised in that compounds corresponding to formula I are produced wherein R' is a $C_1$-$C_4$-alkoxy group or a phenyl-$C_1$-$C_2$-alkoxy group and $R'_2$ is hydrogen or fluorine.

6. Use of compounds corresponding to formula I

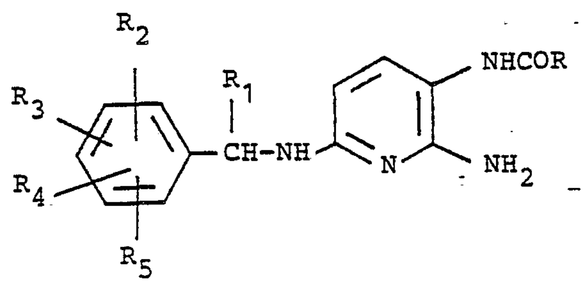

(I)

wherein R represents a $C_1$-$C_4$-alkyl group, a $C_1$-$C_4$-alkoxy group, a phenoxy group a phenyl-$C_1$-$C_2$-alkoxy group, $R_1$ represents hydrogen or a $C_1$-$C_4$-alkyl group and $R_5$ represents hydrogen or a $C_1$-$C_4$-alkyl group and the radicals $R_2$, $R_3$ and $R_4$ are the same or different and represent hydrogen, trifluoromethyl, halogen atoms, $C_1$-$C_4$-alkyl groups, $C_1$-$C_4$-alkylcarbonyl groups, the aminosulphonyl group ($NH_2$—$SO_2$—) or a $C_1$-$C_4$-alkylcarbonylamino group and the acid addition salts thereof for the production of a pharmaceutical composition for the treatment of epilepsy.

7. Use of the compounds corresponding to formula I

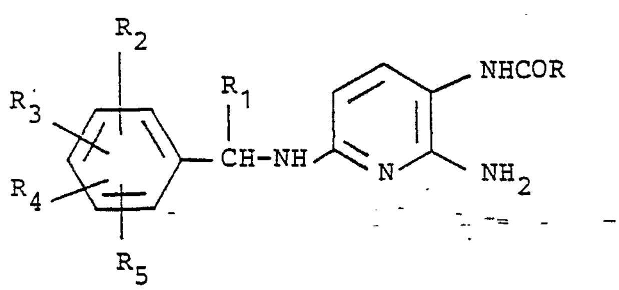

(I)

wherein R is a $C_1$-$C_4$-alkyl group, a $C_1$-$C_4$-alkoxy group, a phenoxy group or a phenyl-$C_1$-$C_2$-alkoxy group, $R_1$ is hydrogen or a $C_1$-$C_4$-alkyl group and $R_2$ is hydrogen, trifluoromethyl, halogen, a $C_1$-$C_4$-alkylcarbonyl group, a $C_1$-$C_4$-alkylcarbonylamino group or an aminosulphonyl group, the radicals $R_3$ and $R_4$ which may be the same or different represent hydrogen, halogen atoms or $C_1$-$C_4$-alkyl groups and $R_5$

is hydrogen or a $C_1$-$C_4$-alkyl group and the acid addition salts thereof for the production of a pharmaceutical composition for the treatment of epilepsy.

8. Use of the compounds corresponding to formula I

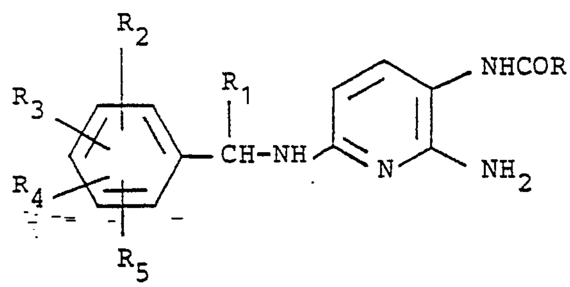

(I)

wherein R is a $C_1$-$C_4$-alkyl group, a $C_1$-$C_4$-alkoxy group, a phenoxy group or a phenyl-$C_1$-$C_2$-alkoxy group, $R_1$ is hydrogen or a $C_1$-$C_4$-alkyl group, $R_2$ is hydrogen, trifluoromethyl, halogen, a $C_1$-$C_4$-alkylcarbonyl group, a $C_1$-$C_4$-alkylcarbonylamino group or an aminosulphonyl group and the radicals $R_3$, $R_4$ and $R_5$ are hydrogen and the acid addition salts thereof for the production of a pharmaceutical composition for the treatment of epilepsy.

9. Use of the compounds corresponding to formula I

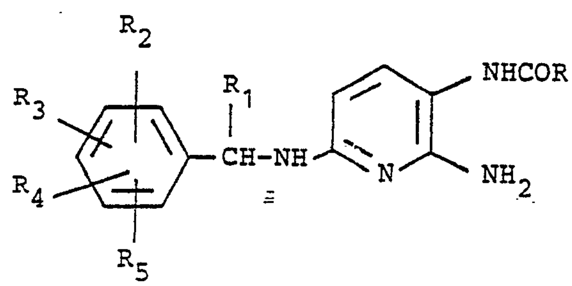

(I)

wherein R is a $C_1$-$C_4$-alkoxy group, $R_2$ is hydrogen, trifluoromethyl, halogen, a $C_1$-$C_4$-alkylcarbonyl group, a $C_1$-$C_4$-alkylcarbonylamino group or an aminosulphonyl group and the radicals $R_1$, $R_3$, $R_4$ and $R_5$ are hydrogen, and the acid addition salts thereof for the production of a pharmaceutical composition for the treatment of epilepsy.

10. Use of the compounds corresponding to formula I

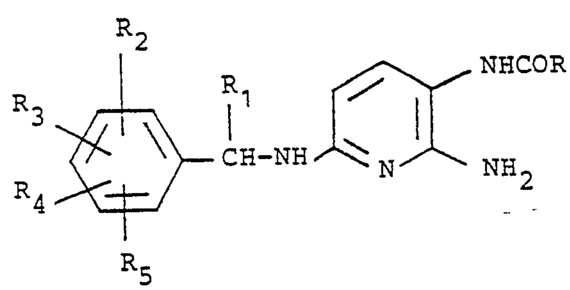

(I)

wherein R is a $C_1$-$C_4$-alkyl group, a phenoxy group or a phenyl-$C_1$-$C_2$-alkoxy group, $R_2$ is hydrogen, trifluoromethyl, halogen, a $C_1$-$C_4$-alkylcarbonyl group, a $C_1$-$C_4$-alkylcarbonyl-amino group or an aminosulphonyl group, and the radicals $R_1$, $R_3$, $R_4$ and $R_5$ are hydrogen, and the acid addition salts thereof for the production of a pharmaceutical composition for the treatment of epilepsy.

11. Use of compounds according to one or more of Claims 1 to 5 for the production of a pharmaceutical composition for the treatment of epilepsy.

**Revendications** (pour les Etats contractants : BE, CH, DE, FR, GB, LI, LU, NL, SE)

1. Composés de formule

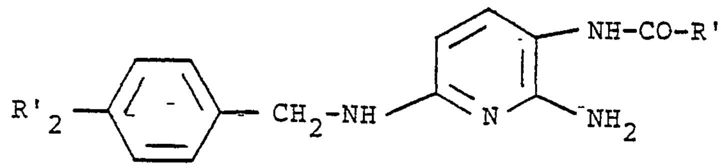

I'

dans laquelle R' représente un groupement alkyle en $C_1$-$C_4$, un groupement alcoxy en $C_1$-$C_4$, un groupement phénoxy ou un groupement phényl-$C_1$-$C_2$-alcoxy et $R'_2$ représente un groupement trifluorométhyle, un groupement $C_1$-$C_4$-alkylcarbonyle, un groupement $C_1$-$C_4$-alkylcarbonylamino ou un groupement aminosulfonyle ($NH_2SO_2$—), $R'_2$ pouvant être également un atome de fluor ou d'hydrogène dans le cas où R' est un groupement alkyle en $C_1$-$C_4$, un groupement phénoxy ou un groupement phényl-$C_1$-$C_2$-alcoxy.

2. Composés selon la revendication 1, dans lesquels R' représente un groupement alkyle en $C_1$-$C_4$, un groupement alcoxy en $C_1$-$C_4$, un groupement phénoxy ou un groupement phényl-$C_1$-$C_2$-alcoxy et $R'_2$ représente un groupement trifluorométhyle, un groupement acétyle, un groupement acétylamino ou un groupement aminosulfonyle, $R'_2$ pouvant être également un atome de fluor ou d'hydrogène dans le cas où R' est un groupement alkyle en $C_1$-$C_4$, un groupement phénoxy ou un groupement phényl-$C_1$-$C_2$-alcoxy.

3. Composés selon la revendication 1, dans lesquels $R'_2$ est un groupement trifluorométhyle.

4. Composés selon la revendication 1, dans lesquels R' est un groupement alcoxy en $C_1$-$C_4$ ou un groupement phénoxy et $R'_2$ est un groupement trifluorométhyle.

5. Composés selon la revendication 1, dans lesquels R' est un groupement alkyle en $C_1$-$C_4$, un groupement phénoxy ou un groupement phényl-$C_1$-$C_2$-alcoxy et $R'_2$ est un atome de fluor ou d'hydrogène.

6. Médicament à activité anti-épileptique, contenant au moins un composé de formule I'

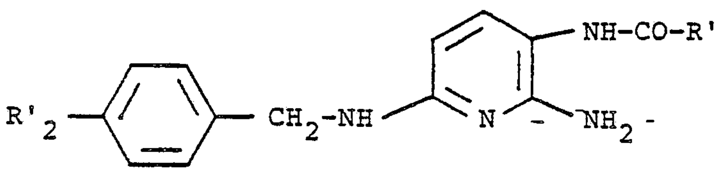

I'

dans laquelle R' représente un groupement alkyle en $C_1$-$C_4$, un groupement alcoxy en $C_1$-$C_4$, un groupement phénoxy ou un groupement phényl-$C_1$-$C_2$-alcoxy et $R'_2$ représente un groupement trifluorométhyle, un groupement $C_1$-$C_4$-alkylcarbonyle, un groupement amino-$C_1$-$C_4$-alkylcarbonylamino ou un groupement aminosulfonyle ($NH_2SO_2$—), $R'_2$ pouvant être également un atome de fluor ou d'hydrogène dans le cas où R' est un groupement alkyle en $C_1$-$C_4$, un groupement phénoxy ou un groupement phényl-$C_1$-$C_2$-alcoxy, ou sel de ce composé formé par addition d'acide.

7. Médicament à activité anti-épileptique selon la revendication 6, caractérisé en ce qu'il contient en outre des excipients et/ou diluants inertes usuels.

8. Procédé de préparation d'un médicament à activité anti-épileptique, caractérisé en ce qu'au moins un composé selon l'une quelconque des revendications 1 à 5 est transformé, avec des excipients ou diluants pharmaceutiques usuels, en un médicament pour le traitement de l'épilepsie.

9. Procédé de préparation de composés selon la revendication 1, caractérisé en ce que :

a) dans un composé de formule

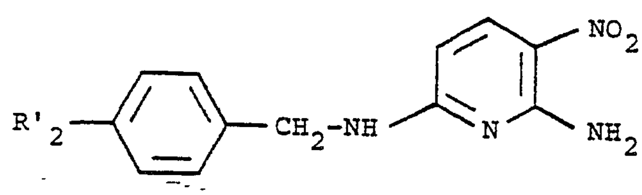

II'

dans laquelle le radical $R'_2$ a les significations données ci-dessus, on réduit le groupement nitro en groupement amino et on introduit par acylation, dans le groupement amino en position 3 ainsi obtenu, le radical R'CO—, R' ayant les significations données ci-dessus, ou

b) on fait réagir un composé de formule générale

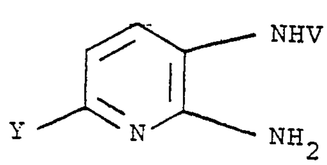

III'

avec un composé de formule générale

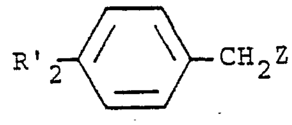

IV'

V dans la formule III' représente un atome d'hydrogène ou le groupement —COR' et Y représentant un groupement amino dans le cas où Z est un atome d'halogène, un groupement amino ou le groupement

—OW

dans lequel W est un atome d'hydrogène, un groupement alkyle inférieur ou un groupement phényle éventuellement substitué par des atomes d'halogène, des groupements nitro ou des groupements méthyle, ou Z pouvant être également un groupement amino dans le cas où Y est un atome d'halogène, un groupement hydroxy ou le groupement OW, avec ou sans solvant, de préférence en présence d'un agent de condensation à température élevée, ou

c) on condense, avec réduction simultanée, un composé de formule générale III', dans lequel Y est le groupement amino et V a les significations données ci-dessus, avec un composé de formule générale

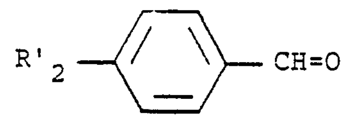

V'

et, dans les composés obtenus par les procédés b) et c), qui ont un groupement amino libre en position 3 du noyau pyridine, on introduit par acylation le radical R'CO— dans ce groupement amino en position 3 et, le cas échéant, on transforme les composés obtenus par les procédés a) à c) en leurs sels d'acides d'addition.

10. Utilisation de composés de formule I

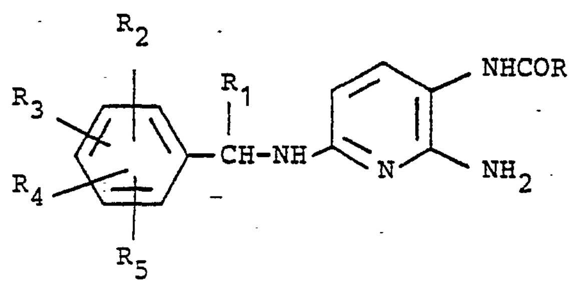

(I)

dans laquelle R représente un groupement alkyle en $C_1$-$C_4$, un groupement alcoxy en $C_1$-$C_4$, un groupement phénoxy ou un groupement phényl-$C_1$-$C_2$-alcoxy, $R_1$ représente un atome d'hydrogène ou un groupement alkyle en $C_1$-$C_4$ et $R_5$ représente un atome d'hydrogène ou un groupement alkyle en $C_1$-$C_4$, et les radicaux $R_2$, $R_3$ et $R_4$ sont identiques ou différents et représentent des atomes d'hydrogène, des radicaux trifluorométhyle, des atomes d'halogènes, des groupements alkyle en $C_1$-$C_4$, des groupements $C_1$-$C_4$-alkylcarbonyle, le groupement aminosulfonyle ($NH_2$—$SO_2$—) ou un groupement $C_1$-$C_4$-alkylcarbonylamino, et de leurs sels d'acides d'addition pour la préparation d'un médicament pour le traitement de l'épilepsie.

11. Utilisation de composés de formule I

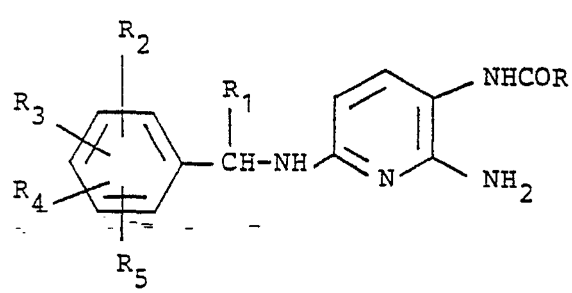

(I)

dans laquelle R est un groupement alkyle en $C_1$-$C_4$, un groupement alcoxy en $C_1$-$C_4$, un groupement phénoxy ou un groupement phényl-$C_1$-$C_2$-alcoxy, $R_1$ est un atome d'hydrogène ou un groupement alkyle en $C_1$-$C_4$ et $R_2$ est un atome d'hydrogène, un groupement trifluorométhyle, un atome d'halogène, un groupement $C_1$-$C_4$-alkylcarbonylamino ou un groupement aminosulfonyle, les radicaux $R_3$ et $R_4$ sont identiques ou différents et représentent des atomes d'hydrogène, des atomes d'halogènes ou des

groupements alkyle en $C_1$-$C_4$ et $R_5$ est un atome d'hydrogène ou un groupement alkyle en $C_1$-$C_4$, et de leurs sels d'acides d'addition pour la préparation d'un médicament pour le traitement de l'épilepsie.

12. Utilisation de composés de formule I

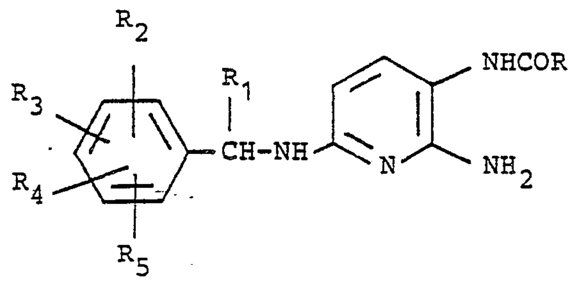

(I)

dans laquelle R est un groupement alkyle en $C_1$-$C_4$, un groupement alcoxy en $C_1$-$C_4$, un groupement phénoxy ou un groupement phényl-$C_1$-$C_2$-alcoxy, $R_1$ est un atome d'hydrogène ou un groupement $C_1$-$C_4$-alcoxy, $R_2$ est un atome d'hydrogène, un groupement trifluorométhyle, un atome d'halogène, un groupement $C_1$-$C_4$-alkylcarbonyle, un groupement $C_1$-$C_4$-alkylcarbonylamino ou un groupement aminosulfonyle et les radicaux $R_3$, $R_4$ et $R_5$ sont des atomes d'hydrogène, et de leurs sels d'acides d'addition pour la préparation d'un médicament pour le traitement de l'épilepsie.

13. Utilisation de composés de formule I

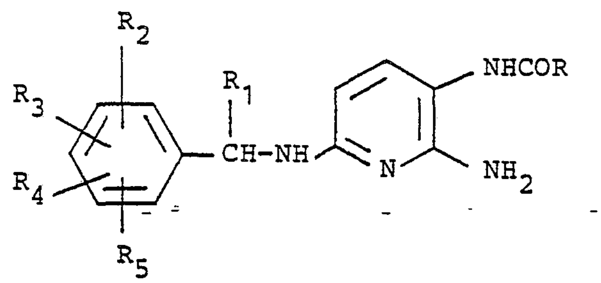

(I)

dans laquelle R est un groupement alcoxy en $C_1$-$C_4$, $R_2$ est un atome d'hydrogène, un groupement trifluorométhyle, un atome d'halogène, un groupement $C_1$-$C_4$-alkylcarbonyle, un groupement $C_1$-$C_4$-alkylcarbonylamino ou un groupement aminosulfonyle et les radicaux $R_1$, $R_3$, $R_4$ et $R_5$ sont des atomes d'hydrogène, et de leurs sels d'acides d'addition pour la préparation d'un médicament pour le traitement de l'épilepsie.

14. Utilisation de composés de formule I

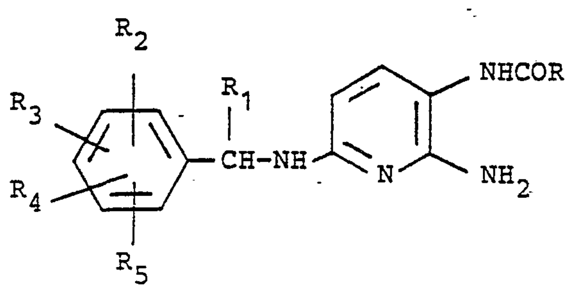

(I)

dans laquelle R est un groupement alkyle en $C_1$-$C_4$, un groupement phénoxy ou un groupement phényl-$C_1$-$C_2$-alcoxy, $R_2$ est un atome d'hydrogène, un groupement trifluorométhyle, un atome d'halogène, un groupement $C_1$-$C_4$-alkylcarbonyle, un groupement $C_1$-$C_4$-alkylcarbonylamino ou un groupement aminosulfonyle et les radicaux $R_1$, $R_3$, $R_4$ et $R_5$ sont des atomes d'hydrogène, et de leurs sels d'acides d'addition pour la préparation d'un médicament pour le traitement de l'épilepsie.

15. Utilisation de composés selon l'une ou plusieurs des revendications 1 à 5 pour la préparation d'un médicament pour le traitement de l'épilepsie.

**Revendications** (pour l'Etat contractant AT)

1. Procédé de préparation de composés de formule

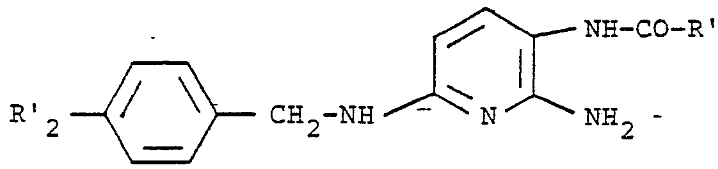

I'

dans laquelle R' désigne un groupement alkyle en $C_1$-$C_4$, un groupement alcoxy en $C_1$-$C_4$, un groupement phénoxy ou un groupement phényl-$C_1$-$C_2$-alcoxy et $R'_2$ représente un groupement trifluorométhyle, un groupement $C_1$-$C_4$-alkylcarbonyle, un groupement $C_1$-$C_4$-alkylcarbonylamino ou un groupement aminosulfonyle ($NH_2SO_2$—), $R'_2$ pouvant être également un atome de fluor ou d'hydrogène dans le cas où R' est un groupement alkyle en $C_1$-$C_4$, un groupement phénoxy ou un groupement phényl-$C_1$-$C_2$-alcoxy, caractérisé en ce que :

a) dans un composé de formule

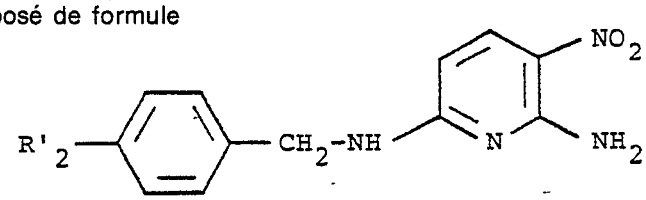

II'

dans laquelle le radical $R'_2$ a les significations données ci-dessus, on réduit le groupement nitro en groupement amino et on introduit par acylation, dans le groupement amino en position 3 ainsi obtenu, le radical R'CO—, R' ayant les significations données ci-dessus, ou

b) on fait réagir, avec ou sans solvant, de préférence en présence d'un agent de condensation, à température élevée, un composé de formule générale

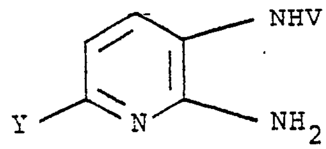

III'

avec un composé de formule générale

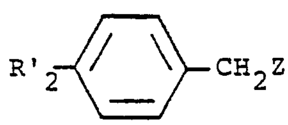

IV'

V dans la formule III' étant un atome d'hydrogène ou le groupement —COR' et Y représentant un groupement amino dans le cas où Z représente un atome d'halogène, un groupement amino ou le groupement

—OW

W étant un atome d'hydrogène, un groupement alkyle inférieur ou un groupement phényle éventuellemnt substitué par des atomes d'halogènes, des groupements nitro ou des groupements méthyle, ou Z pouvant être également un groupement amino dans le cas où Y est un atome d'halogène, un groupe hydroxy ou le groupement OW, ou

c) on condense, avec réduction simultanée, un composé de formule générale III', dans laquelle Y est le groupe amino et V a les significations données ci-dessus, avec un composé de formule générale

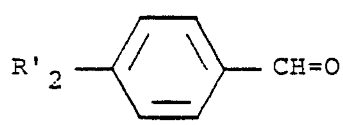

V'

et, dans les composés obtenus par les procédés b) et c), qui ont un groupe amino libre en position 3 du noyau pyridine, on introduit le radical R'CO— par acylation dans ce groupement amino en position 3 et, le cas échéant, on transforme les composés obtenus par les procédés a) à c) en leurs sels d'acides d'addition.

2. Procédé selon la revendication 1, caractérisé en ce qu'on prépare des composés de formule I dans laquelle R' désigne un groupement alkyle en $C_1$-$C_4$, un groupement alcoxy en $C_1$-$C_4$, un groupement phénoxy ou un groupement phényl-$C_1$-$C_2$-alcoxy et $R'_2$ représente un groupement trifluorométhyle, un

groupement acétyle, un groupement acétylamino ou un groupement aminosulfonyle, $R'_2$ pouvant être également un atome de fluor ou d'hydrogène dans le cas où R' est un groupement alkyle en $C_1$-$C_4$, un groupement phénoxy ou un groupement phényl-$C_1$-$C_2$-alcoxy.

3. Procédé selon la revendication 1, caractérisé en ce qu'on prépare des composés de formule I dans laquelle $R'_2$ est un groupement trifluorométhyle.

4. Procédé selon la revendication 1, caractérisé en ce qu'on prépare des composés de formule I dans laquelle R' est un groupement alcoxy en $C_1$-$C_4$ ou un groupement phénoxy et $R'_2$ est un groupement trifluorométhyle.

5. Procédé selon la revendication 1, caractérisé en ce qu'on prépare des composés de formule I dans laquelle R' est un groupement alkyle en $C_1$-$C_4$, un groupement phénoxy ou un groupement phényl-$C_1$-$C_2$-alcoxy et $R'_2$ est un atome d'hydrogène ou de fluor.

6. Utilisation de composés de formule I

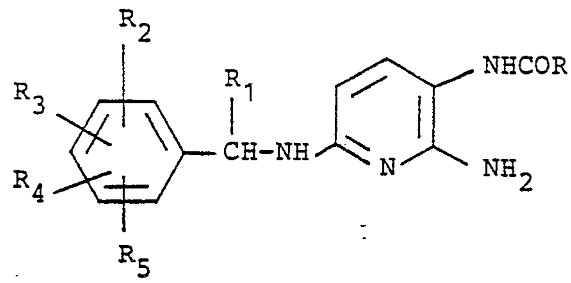

(I)

dans laquelle R représente un groupement alkyle en $C_1$-$C_4$, un groupement alcoxy en $C_1$-$C_4$, un groupement phénoxy ou un groupement phényl-$C_1$-$C_2$-alcoxy, $R_1$ représente un atome d'hydrogène ou un groupement alkyle en $C_1$-$C_4$, $R_5$ représente un atome d'hydrogène ou un groupe alkyle en $C_1$-$C_4$ et les radicaux $R_2$, $R_3$ et $R_4$ sont identiques ou différents et représentent des atomes d'hydrogène, des groupements trifluorométhyle, des atomes d'halogènes, des groupements alkyle en $C_1$-$C_4$, des groupements $C_1$-$C_4$-alkylcarbonyle, des groupements aminosulfonyle ($NH_2$—$SO_2$—) ou des groupements $C_1$-$C_4$-alkylcarbonylamino, et de leurs sels d'acides d'addition pour la préparation d'un médicament pour le traitement de l'épilepsie.

7. Utilisation de composés de formule I

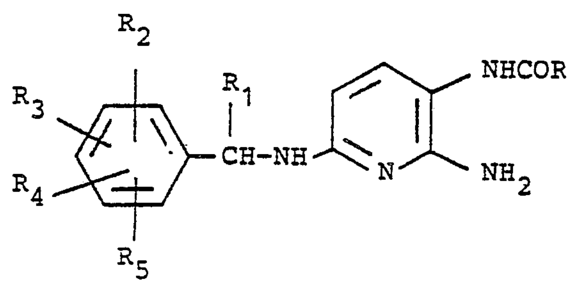

(I)

dans laquelle R est un groupement alkyle en $C_1$-$C_4$, un groupement alcoxy en $C_1$-$C_4$, un groupement phénoxy ou un groupement phényl-$C_1$-$C_2$-alcoxy, $R_1$ est un atome d'hydrogène ou un groupement alkyle en $C_1$-$C_4$ et $R_2$ est un atome d'hydrogène, un groupement trifluorométhyle, un atome d'halogène, un groupement $C_1$-$C_4$-alkylcarbonyle, un groupement $C_1$-$C_4$-alkylcarbonylamino ou un groupement aminosulfonyle, les radicaux $R_3$ et $R_4$ sont identiques ou différents et représentent des atomes d'hydrogène, des atomes d'halogènes ou des groupements alkyle en $C_1$-$C_4$ et $R_5$ est un atome d'hydrogène ou un groupement alkyle en $C_1$-$C_4$, et de leurs sels d'acides d'adition pour la préparation d'un médicament pour le traitement de l'épilepsie.

8. Utilisation de composés de formule I

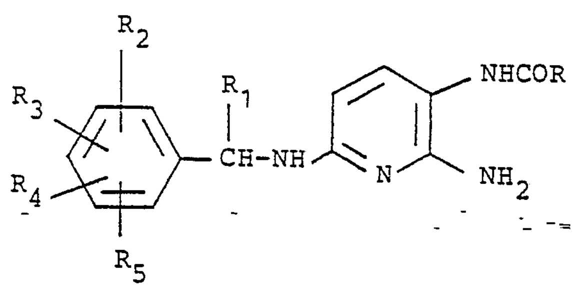

(I)

dans laquelle R est un groupement alkyle en $C_1$-$C_4$, un groupement alcoxy en $C_1$-$C_4$, un groupement phénoxy ou un groupement phényl-$C_1$-$C_2$-alcoxy, $R_1$ est un atome d'hydrogène ou un groupement alkyle en $C_1$-$C_4$, $R_2$ est un atome d'hydrogène, un groupement trifluorométhyle, un atome d'halogène, un groupement $C_1$-$C_4$-alkylcarbonyle, un groupement $C_1$-$C_4$-alkylcarbonylamino ou un groupement aminosulfonyle et les radicaux $R_3$, $R_4$ et $R_5$ sont des atomes d'hydrogène, et leurs sels d'acides d'addition pour la préparation d'un médicament pour le traitement de l'épilepsie.

9. Utilisation de composés de formule I

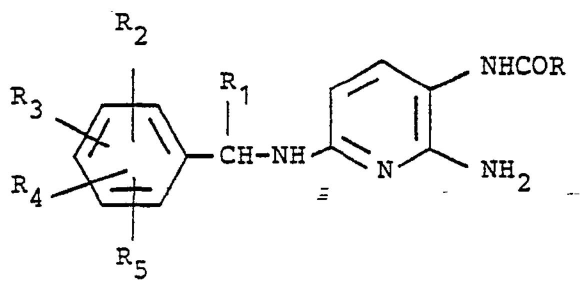

(I)

dans laquelle R est un groupe alcoxy en $C_1$-$C_4$, $R_2$ est un atome d'hydrogène, un groupement trifluorométhyle, un atome d'halogène, un groupement $C_1$-$C_4$-alkylcarbonyle, un groupement $C_1$-$C_4$-alkylcarbonylamino ou un groupement aminosulfonyle et les radicaux $R_1$, $R_3$, $R_4$ et $R_5$ sont des atomes d'hydrogène, et leurs sels d'acides d'addition pour la préparation d'un médicament pour le traitement de l'épilepsie.

10. Utilisation de composés de formule I

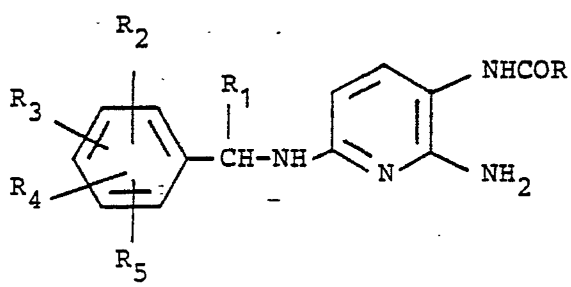

(I)

dans laquele R est un groupement alkyle en $C_1$-$C_4$, un groupement phénoxy ou un groupement phényl-$C_1$-$C_2$-alcoxy, $R_2$ est un atome d'hydrogène, un groupement trifluorométhyle, un atome d'halogène, un groupement $C_1$-$C_4$-alkylcarbonylamino ou un groupement aminosulfonyle et les radicaux $R_1$, $R_3$, $R_4$ et $R_5$ sont des atomes d'hydrogène, et leurs sels d'acides d'addition pour la préparation d'un médicament pour le traitement de l'épilepsie.

11. Utilisation de composés selon l'une ou plusieurs des revendications 1 à 5 pour la préparation d'un médicament pour le traitement de l'épilepsie.